# EUROPEAN PATENT APPLICATION

(11) **EP 2 368 906 A1**
(43) Date of publication of application: **28.09.2011**
(21) Application number: 10012754.7
(22) Date of filing: 29.04.2005
(51) Int. Cl.: C07K 14/47, A61P 35/00, G01N 33/574, A61K 38/17, A61P 43/00

(54) **Fas associated factor 1**

(30) Priority: 29.04.2004 US 566966 P; 21.07.2004 US 590327 P
(62) Divisional of application: 05805203.6
(71) Applicant: SK Corp., Jongrogu Seoul (KR); Ewha Womans University, Seoul (KR)
(72) Inventor: Lee, Kong-Joo, Seoul 120-750 (KR); Kim, Hee.Jung, Seoul 120-750 (KR); Cho, Jeong Woo, daejon 305-712 (KR); Kim, Eunhee, Taejon (KR); Song, Eun Joo, Seoul 130-650 (KR); Maeng, Cheol Young, Seoul 110-110 (KR)
(74) Representative: Fiener, Josef

(57) **Abstract**

The invention refers to a polypeptide fragment of Fas associated Factor 1, which binds to ubiquinated protein. Further, a method for preventing degradation of an ubiquinated protein is described.

## Description

### BACKGROUND OF THE INVENTION

**1. Field of the Invention:**

The invention relates to Fas associated factor 1 (FAF1) and fragments thereof. The invention also relates to the field of cell death. The invention further relates to inhibiting chaperone activity of HSP70 and inhibiting degradation of ubiquinated protein. The invention further relates to a method for screening for a cell death inducer molecule. The invention also relates to cancer diagnostic marker. The invention also relates to cancer treatment. The invention further relates to treating hyperplasia or rapid cell growth. Further the invention relates to treating disease caused by cell death by inhibiting FAF1 activity.

**2. General Background and State of the Art:**

Fas associated factor 1 (FAF1) was first identified as a binding protein to Fas cytoplasmic region in yeast two-hybrid screen in mouse. Transient overexpression of mouse FAF1 (mFAF1) enhances Fas-induced apoptosis in L cells (Chu et al., 1995, Proc. Natl. Acad. Sci. USA 92:11894-11898). Unlike MFAF1, human FAF1 (hFAF1) initiates apoptosis in BOSC23 cells only by transient overexpression. hFAF1 binds to Fas through amino acid sequence 1-201 (Ryu and Kim, 2001, Biochem. Biophys. Res. Commun. 286:1027-1032). Other than Fas, casein kinase 2 subunit (CK2) is the FAF1 binding molecule reported (Kusk et al., 1999, Mol. Cell. Biochem. 191:51-58). hFAF1 is phosphorylated by CK2 on 289 and 291 serine residues and the hFAF1-CK2 complex formation increases when apoptosis occurs (Jensen et al., 2001, Int. J. Biochem. Cell Biol. 33:577-589; Guerra et al., 2001, Int. J. Oncol. 19:1117-1126). Recently, hFAF1 was reported as a member of the Fas-death inducing signaling complex (Fas-DISC) by interacting FADD and caspase-8 (Ryu et al., 2003, J. Biol. Chem. 278:24003-24010).

Unlike other Fas associating proteins, hFAF1 does not contain a death domain but has several homologous domains based on amino acid sequence analysis, two ubiquitin homologous domains (Ubs), one UAS domain homologous with *Caenorhabditis elegans* ORF C281.1, and another domain homologous with proteins involved in the ubiquitin pathway (UBX) (3).

Heat shock protein 70 (Hsp70) participates in folding of newly synthesized proteins, translocation of intracellular proteins, assembly and disassembly of oligomeric protein structures, proteolytic degradation of denatured proteins, and in controlling the activity of regulatory proteins (Hartl, 1996, Nature 381:571-580; Frydman and Höhfeld, 1997, Trends Biochem Sci. 22:87-92; Höhfeld and Jentsch, 1997, EMBO J. 16:6209-6216; Bukau and Horwich, 1998, Cell 92:351-366). As Hsp70 exerts its various roles through binding to various chaperone cofactors or co-chaperones, the fate of substrate proteins is determined by the nature of the co-chaperones. In *Escherichia coli,* the Hsp70 homologue, DnaK, is shown to be assisted by two co-chaperones, DnaJ, which yields the high-substrate-affinity form for substrate binding, and GrpE, which accelerates release of substrates (Liberek et al., 1991, Proc. Natl. Acad. Sci., USA 88:2874-2878; McCarty et al., 1995, J. Mol. Biol. 249:126-137). In mammalian systems, where several co-chaperones have been identified, a homologue of DnaJ, Hsp40/Hdj-1, and Hsc70-interacting protein (Hip/p48) was shown to increase the affinity for substrate protein, thus preventing aggregation of denatured proteins (Minami et al., 1996, J. Biol. Chem. 271:19617-19624; Höhfeld et al., 1995 Cell 83:589-598). Hsc70-Hsp90-organizing protein (Hop/p60/Sti1) interacts with the carboxyl-terminal domain of Hsc70 and serves as an adaptor molecule that forms a Hsc70-Hop-Hsp90 complex, without affecting the chaperone activity of Hsc70 (Demand et al., 1998, Mol. Cell. Biol. 8:2023-2028; Schumacher et al., 1994, J. Biol. Chem., 269:9493-9499; Smith et al., 1993, Mol. Cell. Biol. 13:869-876). BAG-1, which is known to bind to Bcl-2 and thus exerts antiapoptotic activity, binds to ATPase domain of Hsp70 and attenuates Hsc70 chaperone activity (Höhfeld and Jentsch, 1997, EMBO J. 16:6209-6216; Takayama et al., 1997, EMBO J. 16:4887-4896; Zeiner et al., 1997, EMBO J. 16:5483-5490). The C-terminus of Hsc70-interacting protein (CHIP) inhibits its ability to refold non-native proteins (Ballinger et al., 1999, Mol. Cell. Biol. 19:4535-4545). Chap1/PLIC-2 and Chap2/Bat3/Scythe bind to the ATPase domain of a Hsp70 family member (Kaye et al., 2000, FEBS Lett. 467:348-355).

We examined whether hFAF1 regulates the chaperone activity through binding to chaperones in heat shock mediated signaling pathway. Employing immunoprecipitation and identification of the bound proteins using MALDI-TOF MS, we found that hFAF1 is a new Hsc70/Hsp70 binding protein. Transient overexpression of hFAF1 inhibits chaperone activity of Hsp70 indicating that hFAF1 is a co-chaperone of Hsp70. hFAF1 may play a role in the regulation of stress-induced cell death using Hsp70 as a binding partner.

In the ubiquitination pathway, free ubiquitin is activated by the Ub-activating enzyme (E1) through the formation of a thioester between a cysteine in E1 and the C-terminus of Ub. The thioester is subsequently transferred to members of the Ub-conjugating enzyme (E2). Ub-protein ligase (E3) has been shown to be responsible for substrate recognition and for promoting Ub ligation to substrate. These multiubiquitin tagged substrates are recognized and degraded by the 26S proteasome (14). Recently, it was reported that VCP, a member of AAA family (ATPase-associated with different cellular activities), and proteins containing UBA domain, bind to multiubiquitinated substrates and regulate their proteolysis as a post-ubiquitination event (33). Although hFAF1 seems to have multiple functions related to the apoptosis and ubiquitin pathway, its role in this regard is not clear. Because of its multiple ubiquitin related domains, hFAF1 was examined to determine its involvement in the ubiquitin-proteasome pathway. Employing immunochemical techniques combined with mass spectrometric analysis, it was determined that hFAF1 binds to VCP through its C-terminal UBX domain and recruited multiubiquitinated substrates through its N-terminal UBA domain. Transient overexpression of hFAF1 results in the accumulation of ubiquitinated substrates via UBA domain, and inhibits the degradation of these ubiquitinated proteins. hFAF1 plays a role in the ubiquitin-proteasome pathway and regulates the degradation of ubiquitinated proteins in proteasome.

### SUMMARY OF THE INVENTION

In one aspect, the invention is directed to a polypeptide fragment of Fas associated Factor 1, which binds to Hsc70/Hsp70, ubiquinated protein or valosin containing protein.

The polypeptide fragment may bind to Hsc70/Hsp70 and the FAF1 may be human FAF1. Further, the fragment may be from about amino acid position 1 to about 345, from about amino acid position 1 to about 201, from about amino acid position 82 to about 650, or from about amino acid position 82 to about 180. The present invention is also directed to a method of inhibiting chaperone activity of Hsc70/Hsp70, comprising contacting Hsc70/Hsp70 with the above-mentioned polypeptide fragments. The invention is also directed to a method of treating cancer or hyperplasia comprising administering to a subject in need thereof the above-mentioned polypeptide fragment.

The present invention is also directed to a fragment of Fas associated Factor 1, which binds to ubiquinated protein. The fragment may be ubiquitin associated (UBA) domain of Fas associated Factor 1. Further, the fragment may be human Fas associated Factor 1. The fragment of may be from about amino acid position 1 to about 81 of hFAF1. The present invention is also directed to a method for preventing degradation of an ubiquinated protein comprising contacting the ubiquinated protein with the fragment described above. The invention is also directed to a method of treating hyperplasia or cancer comprising administering to a subject in need thereof the above-described composition.

The present invention is also directed to a fragment of Fas associated Factor 1, which binds to valosin containing protein. The fragment may be UBX (ubiquitin regulatory X) domain of Fas associated Factor 1.

The present invention is further directed to a method for diagnosing whether a sample tissue is hyperplasic or cancerous, comprising assaying for the expression of Fas associated factor 1 in the sample tissue and known normal tissue, wherein the comparatively less expression of Fas associated factor 1 in the sample tissue indicates that the sample tissue is hyperplasic or cancerous. The cancer may be carcinoma, melanoma or sarcoma.

These and other objects of the invention will be more fully understood from the following description of the invention, and the referenced drawings attached hereto.

### BRIEF DESCRIPTTON OF THE DRAWINGS

The present invention will become more fully understood from the detailed description given herein below, and the accompanying drawings which are given by way of illustration only, and thus are not limitative of the present invention, and wherein;

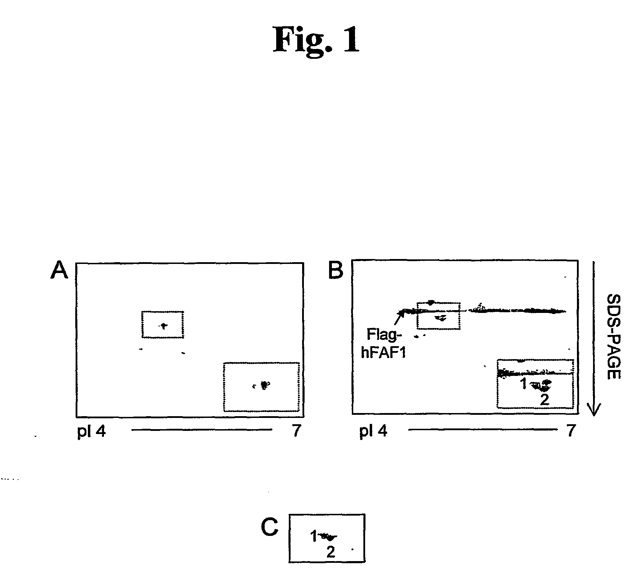

FIGURES 1A-1C show that Flag-hFAF1 interacts with Hsc70 and Hsp70. HEK293T cells were transfected with (A) pFlag-CMV-2 vector and (B) Flag-tagged hFAF1 and labeled with 2 µCi/ml [³⁵S]Methionine. Cells were lysed and immunoprecipitated with anti-Flag M2-agarose crosslinking affinity gel. Precipitates were analyzed by 2D-gel electrophoresis and autoradiographed using BAS2500. Protein spots detected in the autoradiograph were cut out from the corresponding silver stained gel and subjected to in-gel digestion with trypsin and mass peptide fingerprint analyses were conducted. Enlarged figure of dotted rectangle was represented right bottom of each gel. (C) Identified Hsc70/Hsp70 were confirmed by western blot analysis.

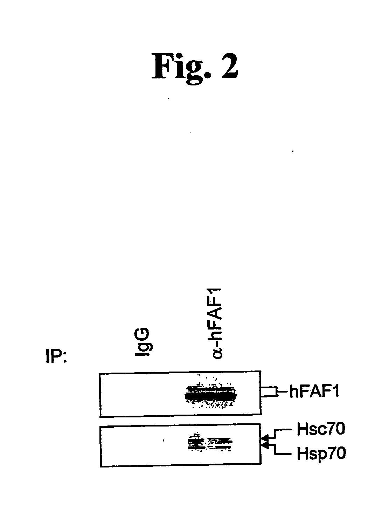

FIGURE 2 shows that Endogenous hFAF1 interacts with endogenous Hsc70/Hsp70. HEK293T cells were lysed and immunoprecipitated with mouse IgG as control (lane 1) and anti-hFAF1 polyclonal antibody (lane 2). Precipitates were analyzed by SDS-PAGE and western blot analysis using anti-hFAF1 polyclonal antibody (upper panel) and anti-Hsc70/Hsp70 monoclonal antibody (lower panel).

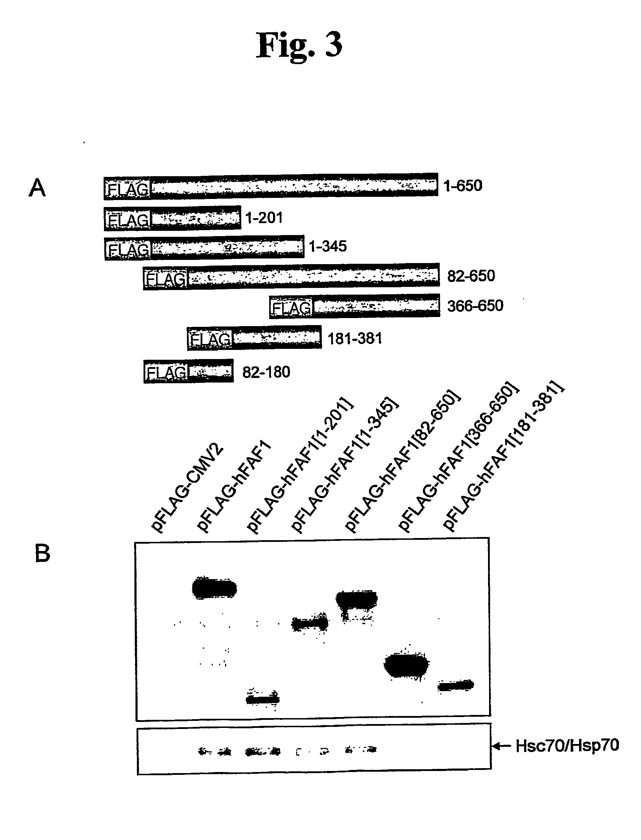

FIGURES 3A-3C show that Hsc70 and Hsp70 interact with the amino acid 82-180 of hFAF1 *in vivo.* (A) Diagram of Flag-tagged hFAF1 fragments. HEK293T cells were transfected with pFlag-CMV-2 vector or pFlag-CMV/hFAF1 fragments shown in (A). Cells were lysed and immunoprecipitated with anti-Flag M2-affinity crosslinking agarose beads (B, C) or with monoclonal anti-Flag antibody and with protein A beads. Precipitates were analyzed by western blot analysis using anti-Flag M2 monoclonal antibody (upper panels of B and C) and anti-Hsc70/Hsp70 antibody (lower panels of B and C).

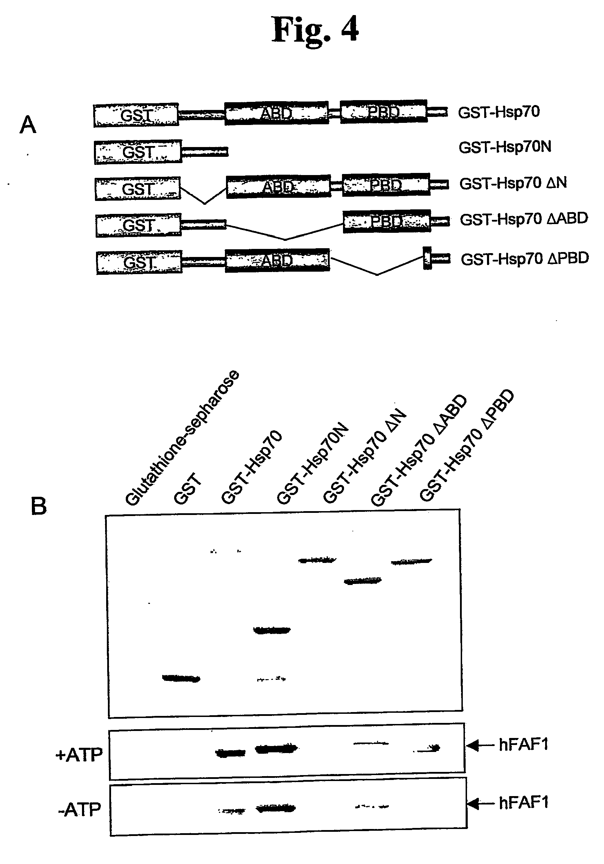

FIGURES 4A-4B show that hFAF1 directly interacts with the N-terminal domain of Hsp70 *in vitro.* (A) Diagram of GST-tagged Hsp70 fragments. (B) Purified recombinant hFAF1 was incubated with GST-Hsp70 deletion mutants immobilized on glutathione-sepharose in presence or absence of 10mM ATP. Precipitates were detected by Coomassie blue staining (B, upper panel) and immunoblotting using anti-hFAF1 polyclonal antibody (B, lower two panels).

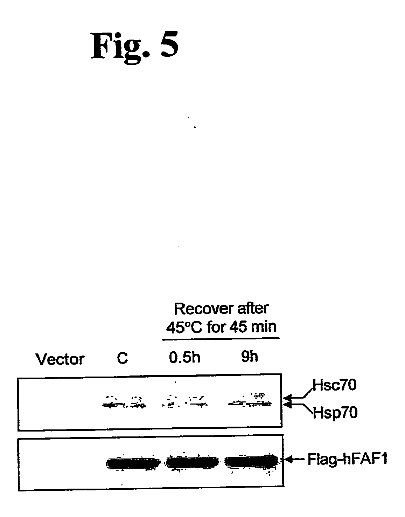

FIGURE 5 shows that heat shock has no effect on the interaction between Hsc701Hsp70 and hFAF1. HEK293T cells were transiently transfected with pFlag-CMV-2 vector or Flag-tagged hFAF1. Cells were heat shocked at 45°C for 45 min or untreated (C) and recovered for the indicated times. At each time point, cells were lysed and immunoprecipitated with monoclonal anti-Flag M2 antibody. Precipitates were analyzed by western blot analysis using anti-Hsc70/Hsp70 (upper panel) and anti-Flag M2 (lower panel) monoclonal antibodies.

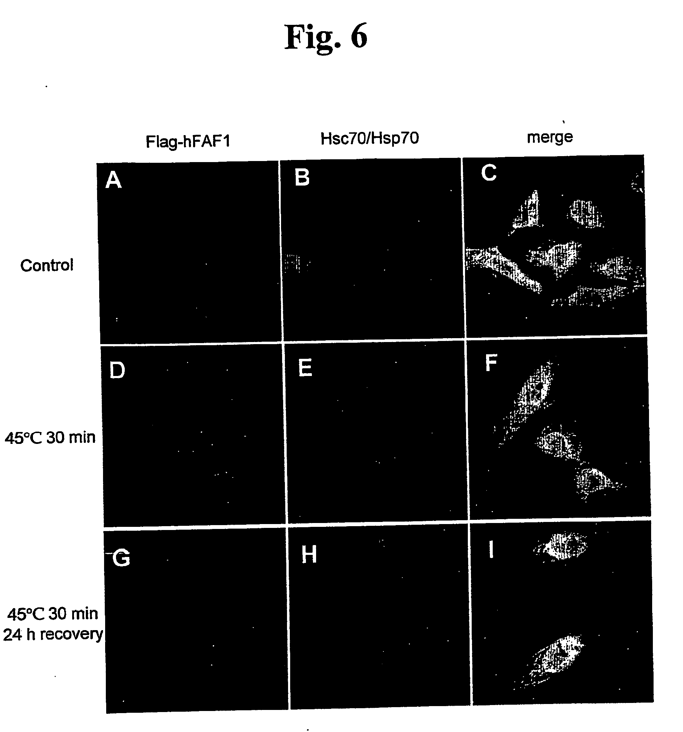

FIGURES 6A-6I show that immunofluorescence analysis of Flag-hFAF1 and Hsp70 localization. Hela cells were transiently transfected with pFlag-CMV-2 vector or pFlag-CMV-2/hFAF1. Cells were immunostained without heat shock (A, B, and C), after heat shock at 45°C for 30 min (D, E, and F), or after recovery period of 24 h (G, H, and I). All cells were stained with a monoclonal antibody to the Hsc70/Hsp70 followed by incubation with Texas red-labeled secondary antibody (red). And then cells were stained with anti-Flag M2 monoclonal antibody labeled with fluorescein isothiocyanate (FTTC) (green). Yellow color in merged figures (C, F, I) means co-localization of red and green fluorescence. The images were made by confocal microscopy.

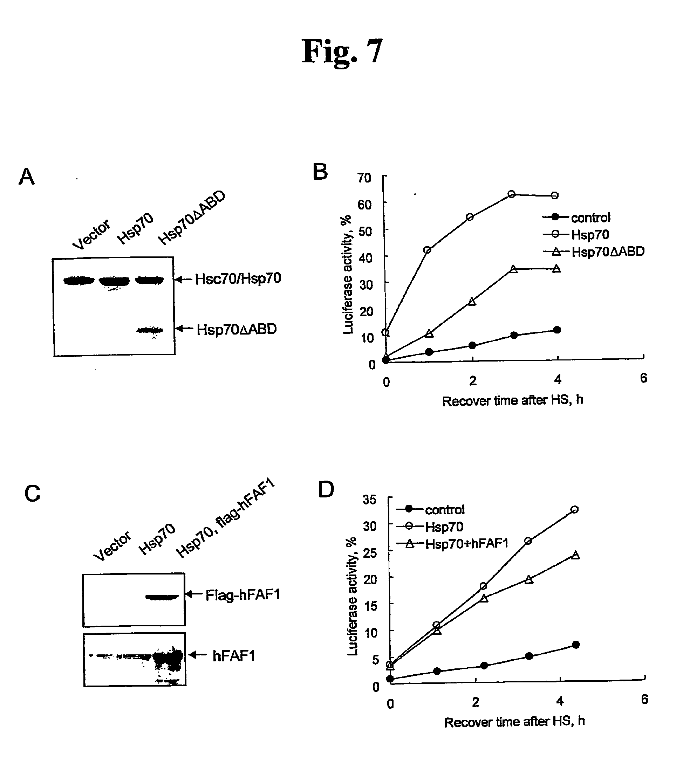

FIGURES 7A-7H show that overexpression of hFAF1 inhibits Hsp70-mediated reactivation of heat-denatured firefly luciferase. HEK293T cells were transiently transfected with pCytluc (encoding cytosolic Luciferase) together with pFlag-CMV-2 vector, pFlag-CMV-2/Hsp70, or pFlag-CMV-2/Hsp70 ΔABD, pFlag-CMV-2/hFAF1, or pFlag-CMV-2/hFAF1ΔN as indicated (A, C, E, G), and detected with Western blot analysis using anti-Hsp70 antibody for Flag-tagged Hsc70/Hsp70, Hsp70ΔABD, and anti-Flag or anti-FAF1 antibody for hFAF1, and hFAF1ΔN expression (A, C, E, G). After pretreatment with cycloheximide (20 µg/ml) for 30 min, luciferase was inactivated by heating the cells at 45°C for 15 min. During a subsequent recovery period at 37°C, samples were assayed for luciferase activity (B, D, F, H).

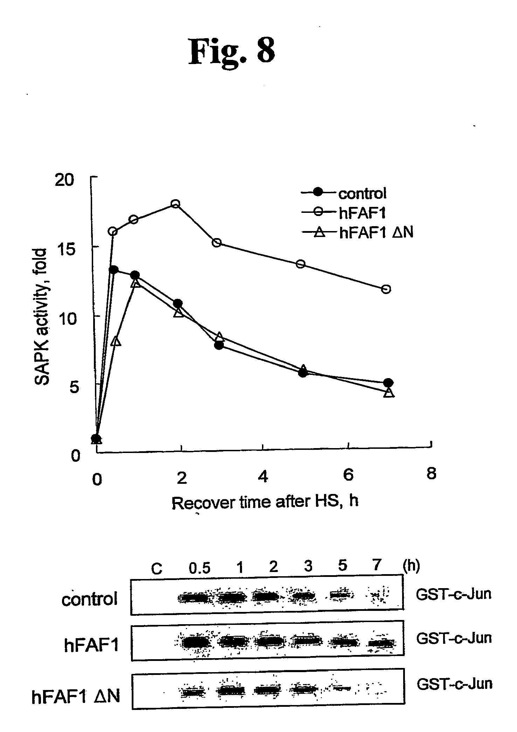

FIGURES 8A-8B show that overexpression of Flag-hFAF1 accelerates heat shock induced SAPK/JNK activation. HEK293T cells were transiently transfected with pFlag-CMV-2 vector, pFlag-CMV-2/hFAF1, or pFlag-CMV-2/hFAF1ΔN and exposed to heat shock at 45°C for 45 min. SAPK/JNK activities were measured after indicated recovery times.

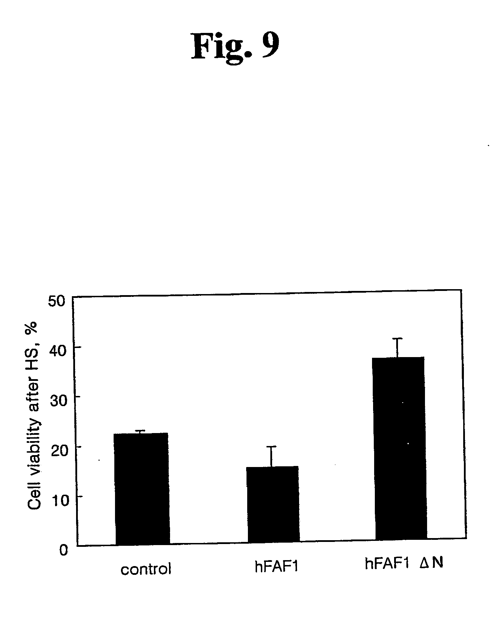

FIGURE 9 shows that overexpression of Flag-hFAF1 increases heat shock induced cell death. HEK293T cells were transiently transfected with pFlag-CMV-2 vector, pFlag-CMV-2/hFAF1, or pFlag-CMV-2/hFAF1ΔN and exposed to heat shock at 45°C for 90 min. After recovery at 37°C for 48 h, the percentage of viable cells was determined by trypan blue dye exclusion assay.

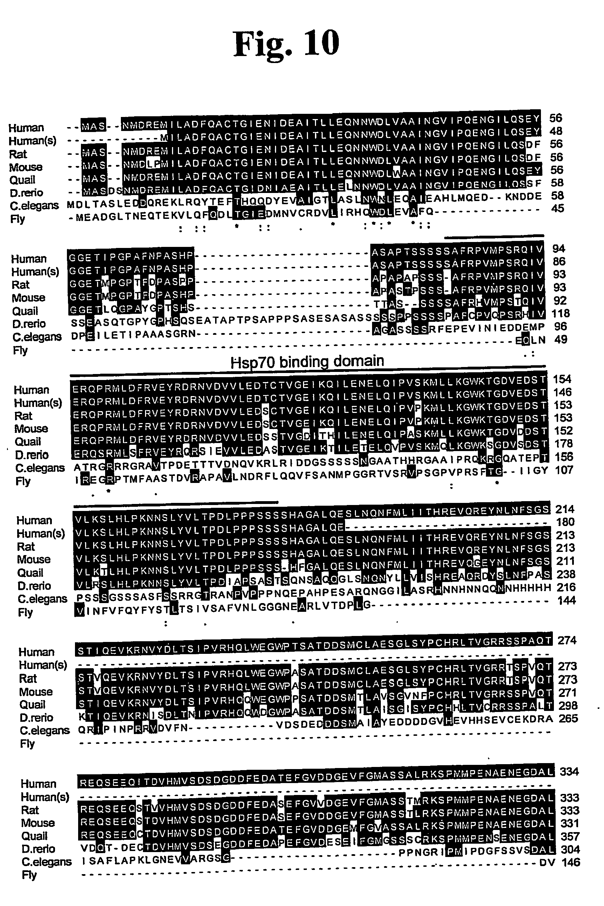

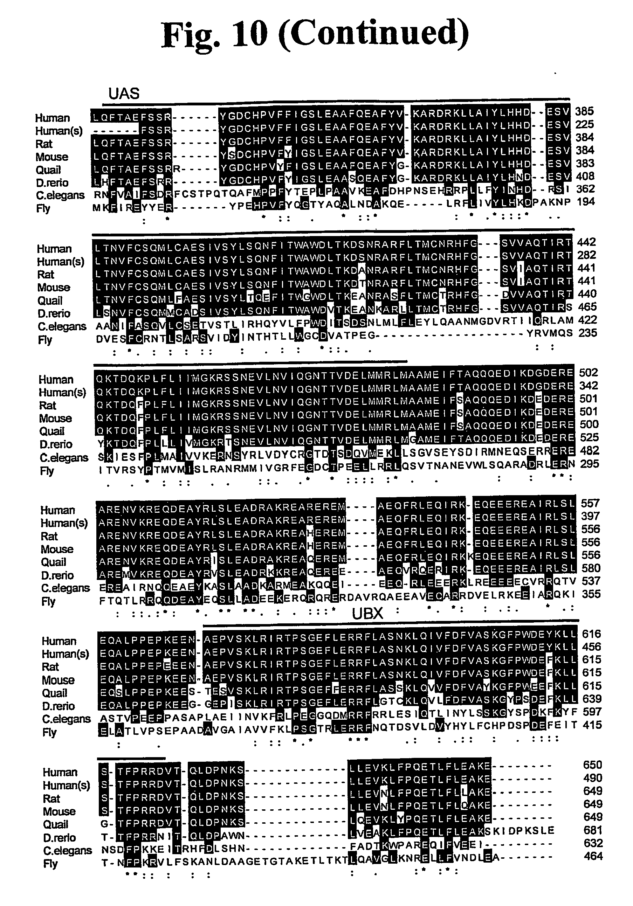

FIGURE 10 shows comparison of amino acid sequences of human, short form of human, rat, mouse, quail, *D. rerio, C*. *elegans,* and fly FAF1 using ClustalW at EBI. Gaps are introduced and represented by dashes, asterisk: the residues in that column are identical in all sequences in the alignment; double dots: conserved substitutions have been observed; one dot: semi-conserved substitutions have been observed; blocks: identical amino acids to hFAF1.

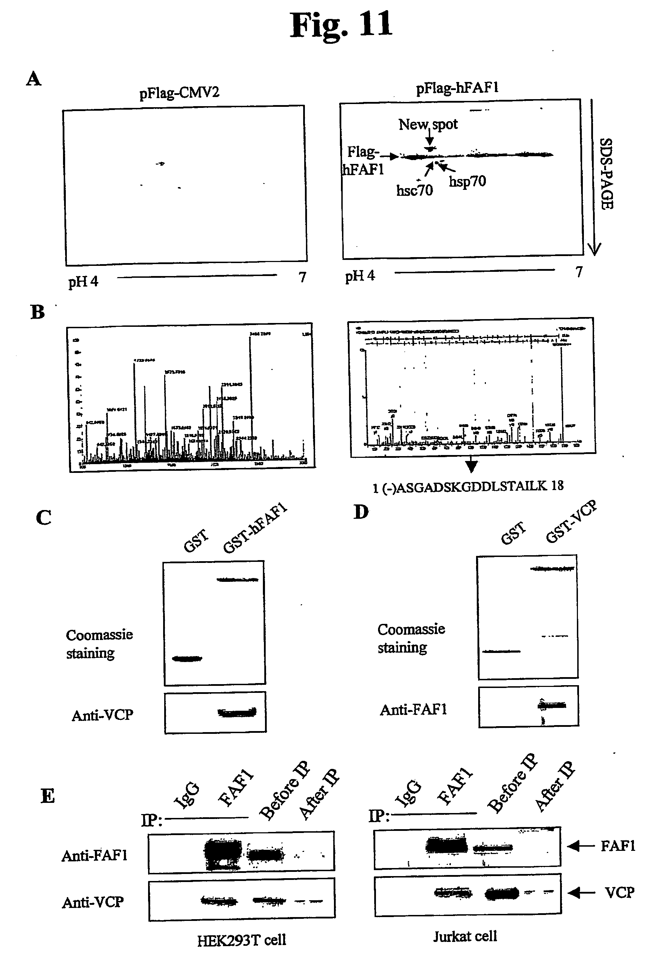

FIGURES 11A-11E show that hFAF1 interacts with VCP *in vitro* and *in vivo.* (A) HEK293T cells transfected with pFlag-CMV-2 vector and pFlag-hFAF1 were labeled with 1 µCi/mL [³⁵S]Methionine. Immunoprecipitation in cell lysates was carried out with anti-Flag M2 agarose crosslinking affinity beads (Sigma). Precipitates were analyzed by 2D-gel electrophoresis and autoradiographed using BAS2500. (B) Protein spots detected in the autoradiograph were cut out from the corresponding silver stained gel and subjected to in-gel digestion with trypsin. Mass peptide fingerprint analyses were conducted by MALDI-TOF MS (left spectrum) and peptide were sequenced using ESI-q-TOF tandem MS (right spectrum). (C, D) GST-hFAF1 (C) and GST-VCP (D) were immobilized on glutathione beads and incubated with purified recombinant VCP (C) and purified recombinant hFAF1 (D). Binding proteins were detected on 10% SDS-PAGE with coomassie staining and immunoblotted by anti-VCP antibody (C) and anti-FAFI antibody (D). (E) For the interaction between endogenous FAF1 and VCP, HEK 293T cell and Jurkat cell lysates were immunoprecipitated with mouse IgG control (lane 1) and anti-FAF1 polyclonal antibodies (lane 2). The amount of endogenous FAF1 and VCP in 10 % of cell lysates before immunoprecipitation was compared with the amounts after immunoprecipitation. Immunoprecipitates were analyzed by western blotting using anti-FAF1 and anti-VCP antibodies.

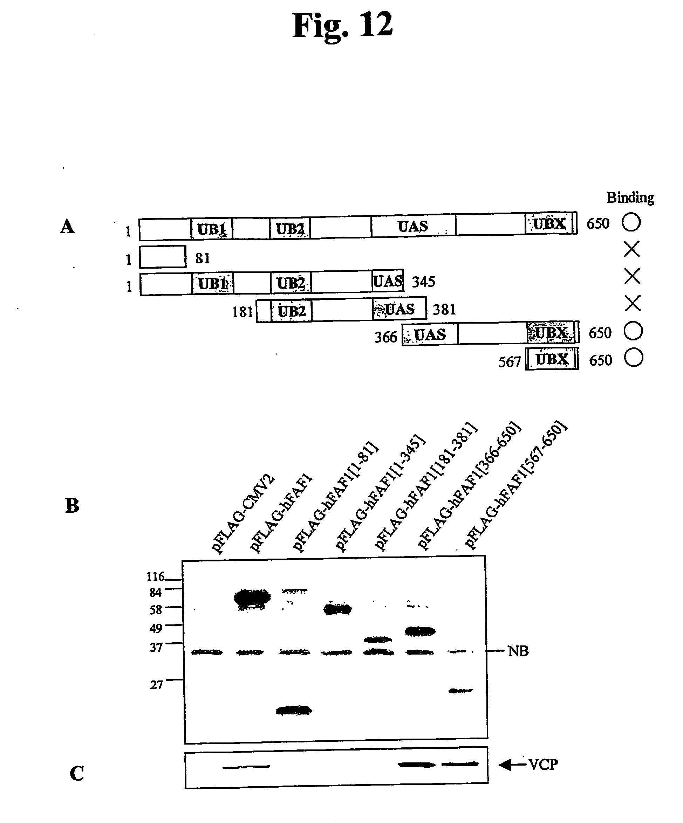

FIGURES 12A-12C show that hFAF1 interacts with VCP via UBX domain. (A) Diagram of various Flag-hFAF1 truncates. UB1 and UB2, ubiquitin homologous domains; UAS domain, homologous with *Caenorhabditis elegans* ORF C281.1; UBX, domain present in ubiquitin-regulatory proteins. (B, C) HEK293T cells transfected with pFlag-CMV-2 vector or truncated Flag-hFAF1 were lysed and immunoprecipitated with monoclonal anti-Flag M2 agarose crosslinking affinity gel (Sigma). Precipitates were analyzed by western blot using anti-Flag (B) and anti-VCP antibodies (C). NB indicates nonspecific protein band.

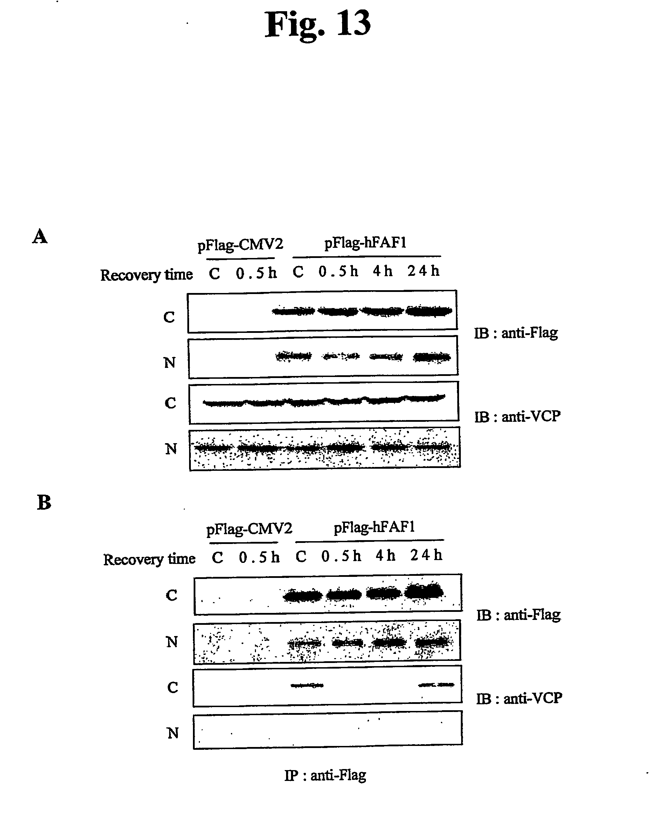

FIGURES 13A-3B show that hFAF1 interacts with VCP in cytosol. HEK293T cells transiently transfected with pFlag-CMV-2 vector or Flag-hFAF1 were heat shocked at 45°C for 45 min and recovered for the indicated times. C indicates untreated cells. At each time point, cells were fractionated into cytosolic (C) and nuclear fraction (N), and western blotted with anti-Flag and anti-VCP antibodies (A). For examining the binding, each fraction was immunoprecipitated with monoclonal anti-Flag M2 agarose crosslinking affinity gel and precipitates were analyzed by western blot analysis using anti-Flag and anti-VCP antibodies (B).

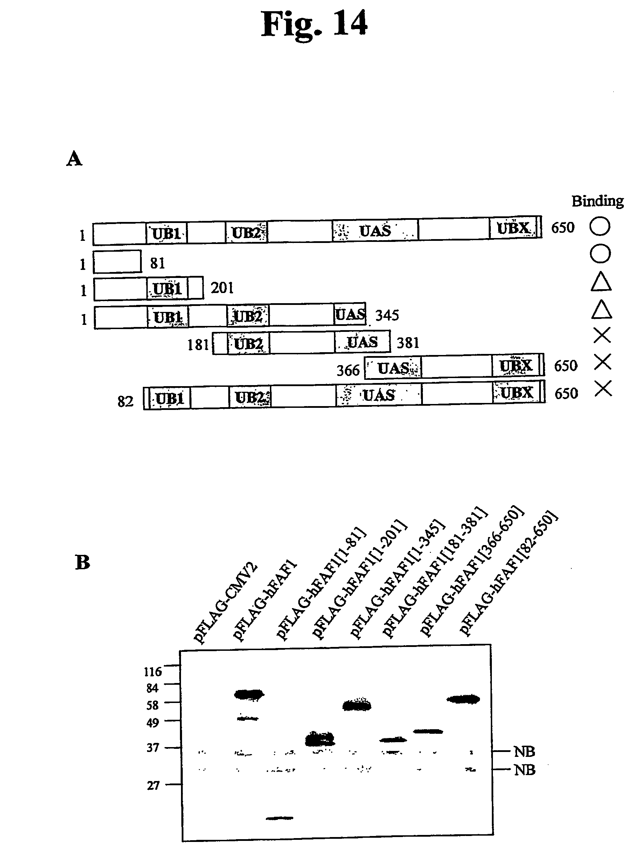

FIGURES 14A-14C show that hFAF1 binds to multiubiquitinated proteins *in vivo.* (A) Diagram of various Flag-hFAF1 truncates. (B, C) HEK293T cells transfected with pflag-CMV-2 vector or Flag-hFAF1 truncates were lysed and immunoprecipitated with monoclonal anti-Flag M2 agarose crosslinking affinity gel (Sigma). Precipitates were analyzed by western blot using anti-Flag (B) and anti-ubiquitin antibodies (C). NB indicates nonspecific band and UBn indicates multiubiquitinated proteins.

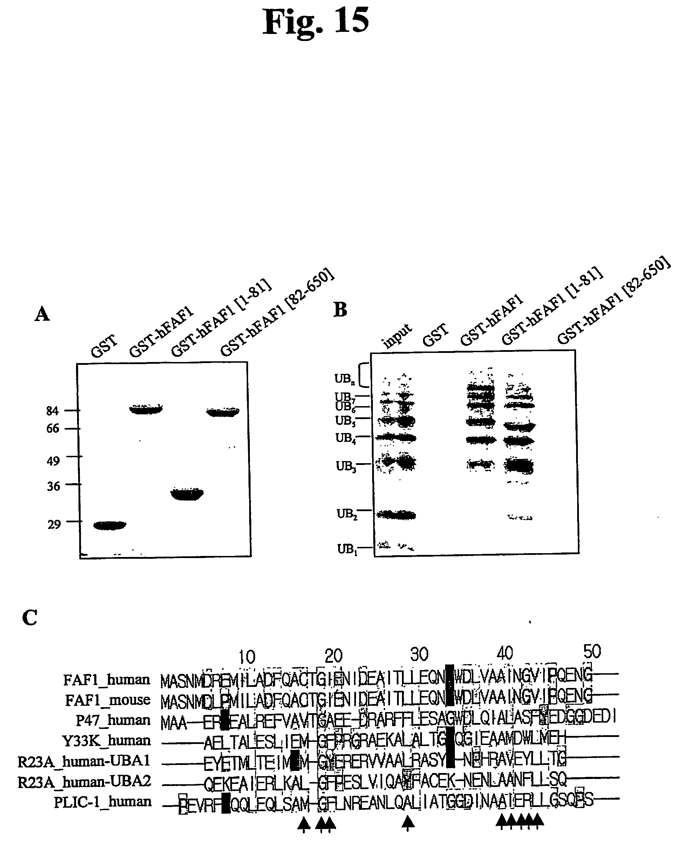

FIGURES 15A-15C show that hFAF1 binds to multiubiquitinated proteins *in vitro.* (A, B) Polyubiquitin chains (Ub₂₋₇) were incubated with GST, GST-hFAF1, GST-hFAF1[1-81] and GST-hFAF1[82-65 proteins immobilized on glutathione-sepharose *in vitro.* Various GST proteins immobilized to glutathione beads were detected on 10% SDS-PAGE with coomassie staining (A) and polyubiquitin chains associated with GST-hFAF1 was detected by immunoblotting using monoclonal anti-ubiquitin antibodies (B). (C) Analysis was performed using program ClustalW (http://www.ebi.ac.uk/clustalw/) and program Jalview (http://www.ebi.ac.uk/)Jalview). Arrows indicate the amino acid residues occupying the conserved positions on hydrophobic surface patch of UBA domain (25). FAF1_human (FAS-associated factor 1 isoform a), NP_008982; FAF1_mouse (FAS-associated factor 1), P54731; P47_human (p47 protein isoform a), NP_057227; Y33K_human (UBA/UBX 33.3 kDa protein), Q04323; R23A_human (UV excision repair protein RAD23 homolog A), P54725; PLIC-1_human (PLIC-1), AAG02473.

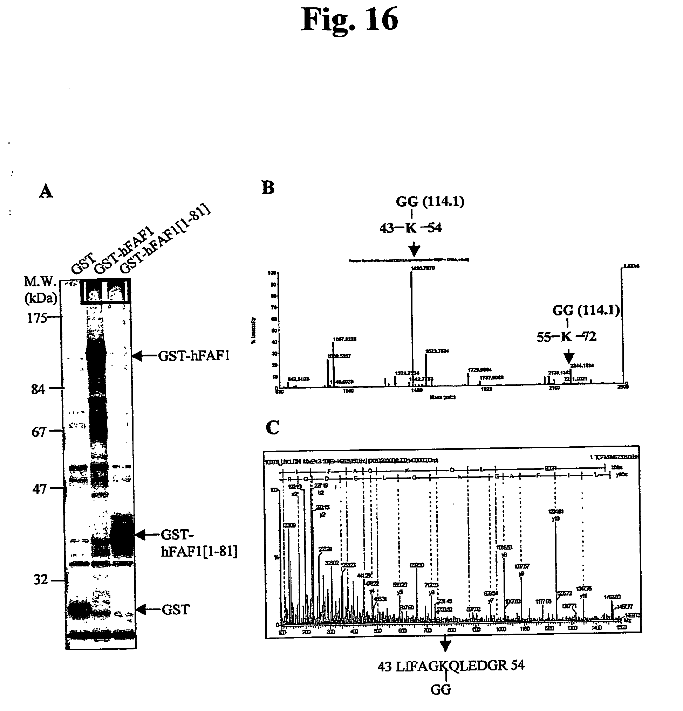

FIGURES 16A-6C show that hFAF1 binds to Lys48-linked and Lys63-linked ubiquitinated proteins via UBA domain. GST, GST-hFAF1 and GST-hFAF1[1-81] proteins were immobilized on glutathione beads, incubated with HEK293T cell lysates and then detected on 10% SDS-PAGE with silver staining (A). High molecular weight proteins indicated as box in (A) were in gel digested with trypsin and analyzed with MALDI-TOF MS (B). Arrows in (B) indicate ubiquitin peptides containing the isopeptide bond linkage through Lys48 (m/z, 1460.7870) and Lys63 (m/z, 2244.1814). The peptide linked through Lys48 was identified by sequencing using ESI-q-TOF tandem MS and amino acid sequence, LIFAGK(-GG)QLEDGR, was determined (C).

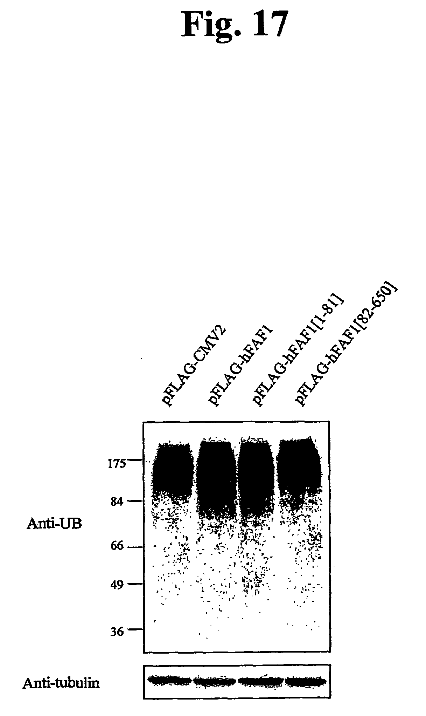

FIGURE 17 shows overexpression of hFAF1 accumulates multiubiquitinated proteins. HEK293T cells transfected with pFlag-CMV-2 vector, Flag-hFAF1, Flag-hFAF1[1-81] and Flag-hFAF1[82-650] were separated on 10% SDS-PAGE and the multiubiquitinated proteins were detected with western analysis using anti-ubiquitin antibody (upper panel). Protein concentrations were normalized with anti-tubulin antibody (lower panel).

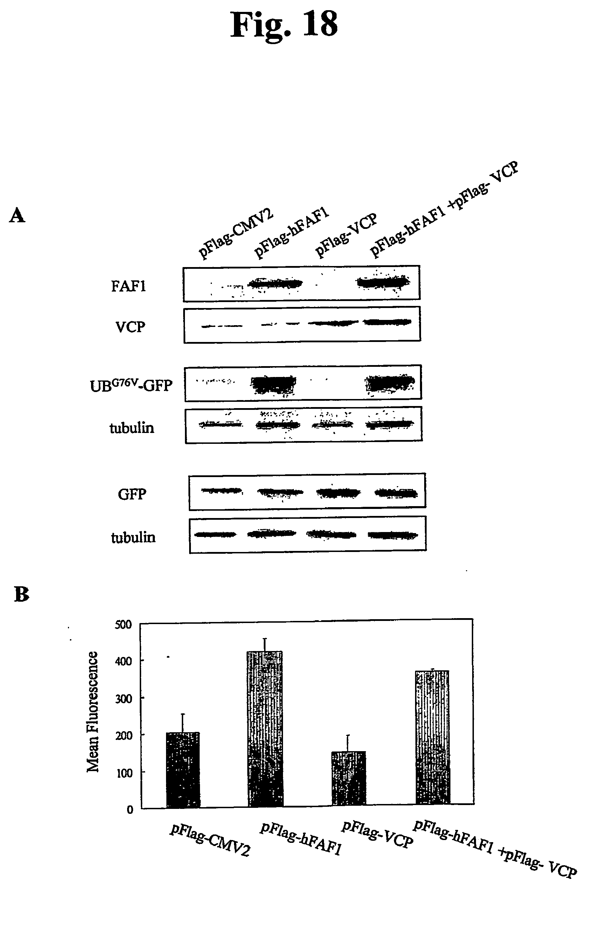

FIGURES 18A-18D show that hFAF1 inhibits ubiquitin-dependent protein degradation via UBA domain. 200 ng of Db^{G76V}-GFP or EGFP-N1 vector was cotransfected with 1 µg of pFlag-CMV-2 vector, Flag-hFAF1, Flag-VCP and mixture of Flag-hFAF1 and Flag-VCP in HEK293T cells seeded in 35 mm dishes at a density of 2 x 10⁵ cells one day before (A, B). 200 ng of Ub^{G76V}-GFP or EGFP-N1 vector was cotransfected with 1 µg of pFlag-CMV-2 vector, Flag-hFAF1, Flag-hFAF1[1-81], Flag-hFAF1[1-345] and Flag-hFAF1[82-650] in HEK293T cells seeded in 35 mm dishes at a density of 2 x 10⁵ cells one day before (C, D). At 24 h after transfection, cells were harvested and divided two portions. One is used for western analysis (A, C) and the other is used for FACS analysis (B, D). (A, C) For confirming protein levels of hFAF1, VCP and truncated hFAF1, cells were lysed, separated in SDS-PAGE and immunoblotted using anti-FAF1 (A), anti-VCP (A) and anti-Flag antibodies (C). In case of Flag-hFAF1 [1-81], western membrane was exposed for a long time (C, Anti-Flag (L.M)) because transfection efficiency was lower than other truncates. The level of Ub^{G76V}-GFP or EGFP-NL vector was detected using anti-GFP antibody and protein concentration was normalized with anti-tubulin antibody. (B, D) Fluorescence of harvested cells was measured by FACS flow cytometer (Beckton & Dickinson) and fluorescence data were analyzed with the Cellquest Software. Their mean fluorescence intensity was shown as mean ± S.D from three independent experiments (P value <0.05).

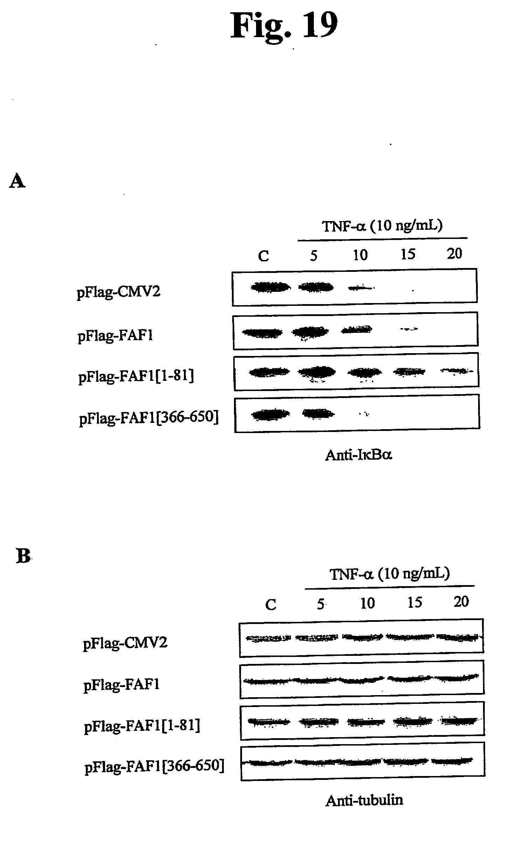

FIGURES 19A-19B show that hFAF1 interferes with degradation of endogenous ubiquitinated substrate. HEK293T cells transfected with pFlag-CMV-2 vector, Flag-hFAF1, Flag-hFAF1[1-81] and Flag-hFAF1[366-650] were exposed to 10 ng/mL of TNF-α for 5, 10, 15 and 20 min at 24 h after transfection. Cells were lysed, separated in 10 % SDS-PAGE and immunoblotted using IκBα antibody (A). C indicates untreated cells. Protein concentration was normalized with anti-tubulin antibody (B).

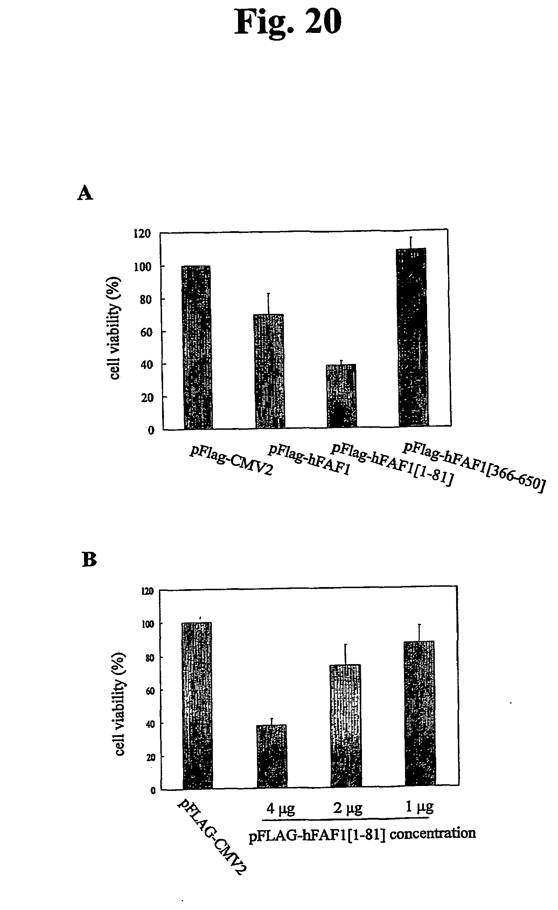

FIGURES 20A-20B show effects of hFAF1 overexpression on cell death. (A) Jurkat cells were transfected with pFlag-CMV-2 vector, Flag-hFAF1, Flag-hFAF1 [1-81] and Flag-hFAF1 [366-650] using Amaxa electroporator. At 24 h after transfection, cell viability was assessed using MTT conversion assay. The relative viability was shown as mean ± S.D from three independent experiments (P value <0.05). (B) Jurkat cells were transfected with 1, 2, 4 µg of Flag-hFAF1[1-81] using Amaxa electroporator. At 24 h after transfection, cell viability was assessed using MTT conversion assay. The relative viability was shown as mean ± S.D from three independent experiments.

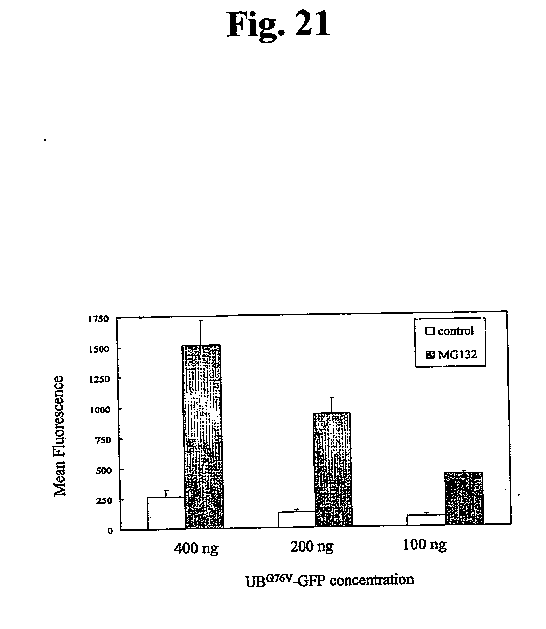

FIGURE 21 shows the effects of proteasome inhibitor on degradation of Ub^{G76V}-GFP. HEK293T cells transiently transfected with 100, 200, 400 ng of Ub^{G76V}-GFP were incubated for 15 h with or without 10 µM MG132 (Calbiochem). At 24 h after transfection, fluorescence of harvested cells was measured by FACS flow cytometer (Beckton & Dickinson) and fluorescence data were analyzed with the Cellquest Software. Their mean fluorescence intensity was shown as mean ± S.D from three independent experiments.

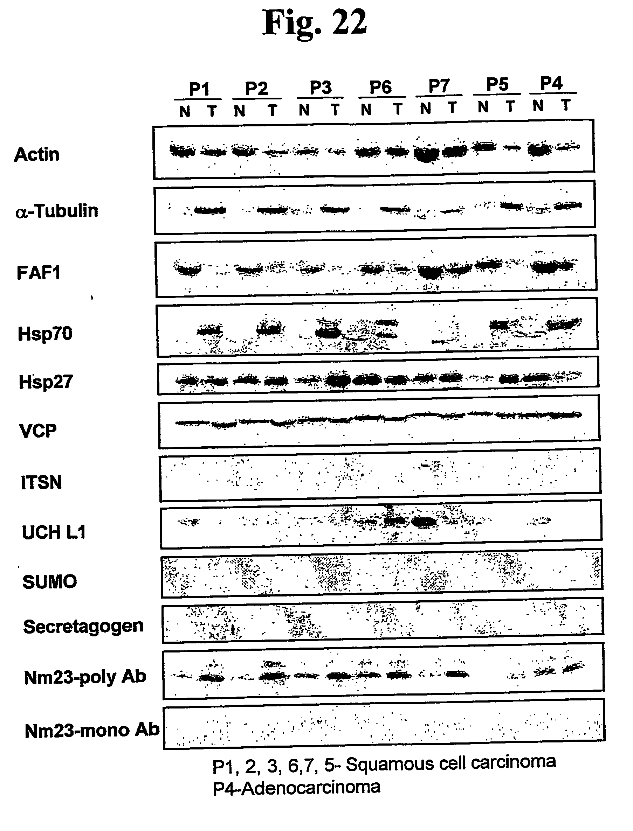

FIGURE 22 shows a Western blot panel of various indicated proteins that are expressed in "N" normal tissue, and "T" ovarian tumor. P1, P2, P3, P6, P7, and P5 represent squamous cell carcinoma; P4 represents adenocarconoma.

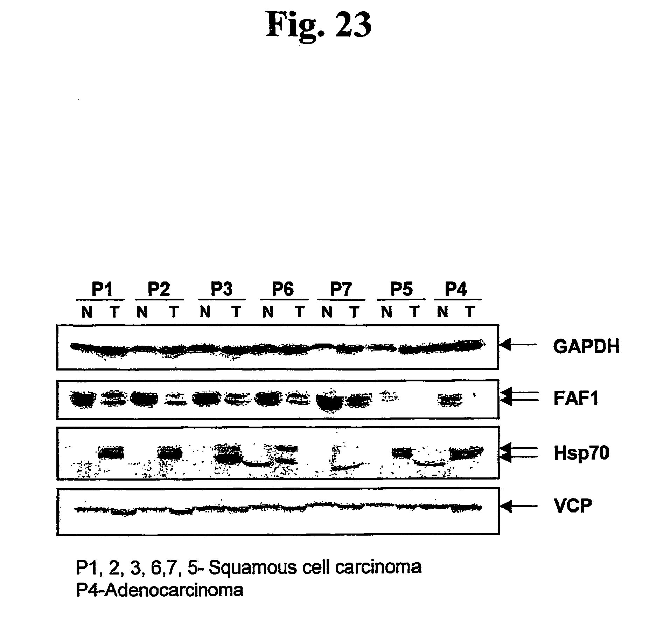

FIGURE 23 shows a Western blot panel of various indicated proteins that are expressed in "N" normal tissue, and "T" ovarian tumor. P1, P2, P3, P6, P7, and P5 represent squamous cell carcinoma; P4 represents adenocarconoma.

### DETAILED DESCRIPTION OF THE PREFERRED EMBODIMENTS

In the present application, "a" and "an" are used to refer to both single and a plurality of objects.

As used herein, "about" or "substantially" generally provides a leeway from being limited to an exact number. For example, as used in the context of the length of a polypeptide sequence, "about" or "substantially" indicates that the polypeptide is not to be limited to the recited number of amino acids. A few amino acids add to or subtracted from the N-terminus or C-terminus may be included so long as the functional activity such as its binding activity is present

As used herein, administration "in combination with" one or more further therapeutic agents includes simultaneous (concurrent) and consecutive administration in any order.

As used herein, "amino acid" and "amino acids" refer to all naturally occurring L-α-amino acids. This definition is meant to include norleucine, ornithine, and homocysteine.

As used herein, in general, the term "amino acid sequence variant" refers to molecules with some differences in their amino acid sequences as compared to a reference (e.g. native sequence) polypeptide. The amino acid alterations may be substitutions, insertions, deletions or any desired combinations of such changes in a native amino acid sequence.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Also included within the scope of the invention are proteins or fragments or derivatives thereof which exhibit the same or similar biological activity and derivatives which are differentially modified during or after translation, e.g., by glycosylation, proteolytic cleavage, linkage to an antibody molecule or other cellular ligand, and so on.

Insertional variants are those with one or more amino acids inserted immediately adjacent to an amino acid at a particular position in a native amino acid sequence. Immediately adjacent to an amino acid means connected to either the α-carboxy or α-amino functional group of the amino acid.

Deletional variants are those with one or more amino acids in the native amino acid sequence removed. Ordinarily, deletional variants will have one or two amino acids deleted in a particular region of the molecule.

As used herein, "Hsc70/Hsp70 binding polypeptide or fragment" refers to a FAF1 polypeptide or fragment that specifically binds to Hsc70/Hsp70 and has at least about 70%, 75%, 80%, 85%, 90%, 95%, 96%, 97%, 98%, 99% or 100% identity to the amino acid region 82 to 180 of human FAF1.

As used herein, "ubiquitinated protein binding polypeptide or fragment" refers to a FAF1 polypeptide or fragment that specifically binds to ubiquitinated protein, preferably multiubiquinated protein and has at least about 70%, 75%, 80%, 85%, 90%, 95%, 96%, 97%, 98%, 99% or 100% identity to the amino acid region 1 to 81 of human FAF1.

As used herein, "valosin containing protein binding polypeptide or fragment" refers to a FAF1 polypeptide or fragment that specifically binds to ubiquitinated protein, preferably multiubiquitinated protein and has at least about 70%, 75%, 80%, 85%, 90%, 95%, 96%, 97%, 98%, 99% or 100% identity to the amino acid region that is 80 amino acids beginning at the carboxy-end of human FAF1.

Applicants for the first time discovered that FAF1 binds to Hsc70/Hsp70, ubiquitinated protein and valosin containing protein, and thus it would be within the purview of a person of skill in the art to make certain variations to the FAF1 sequence, which retains the capability of binding to these proteins. In particular, various constructs may be created to form a non-functional complex with these proteins and a variety of FAF1 sequences may be used to make such as construct

As used herein, "carriers" include pharmaceutically acceptable carriers, excipients, or stabilizers which are nontoxic to the cell or mammal being exposed thereto at the dosages and concentrations employed. Often the pharmaceutically acceptable carrier is an aqueous pH buffered solution. Examples of pharmaceutically acceptable carriers include without limitation buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{®}, polyethylene glycol (PEG), and PLUROIVICS^{®}.

As used herein, "covalent derivatives" include modifications of a native polypeptide or a fragment thereof with an organic proteinaceous or non-proteinaceous derivatizing agent, and post-translational modifications. Covalent modifications are traditionally introduced by reacting targeted amino acid residues with an organic derivatizing agent that is capable of reacting with selected sides or terminal residues, or by harnessing mechanisms of post-translational modifications that function in selected recombinant host cells. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions.

As used herein, "effective amount" is an amount sufficient to effect beneficial or desired clinical or biochemical results. An effective amount can be administered one or more times. For purposes of this invention, an effective amount of an inhibitor compound is an amount that is sufficient to palliate, ameliorate, stabilize, reverse, slow or delay the progression of the disease state. In a preferred embodiment of the invention, the "effective amount" is defined as an anti-tumor amount of a compound capable of binding to Hsc70/Hsp70 and ubiquitinated proteins. In other embodiments, "effective amount" may be that amount of FAF1 polypeptide that binds to and sequesters Hsc70msp70 and/or ubiquitinated proteins to render them unavailable for further reaction. In yet another embodiment of the invention, "effective amount" may refer to the amount of FAF1 fragment or polypeptide that is sufficient to bind and sequester Hsc70/Hsp70 and/or ubiquitinated proteins and to render them inactive to further reaction in the instance where cancer is desired to be treated.

As used herein, "fragment" refers to a part of a polypeptide, which retains usable and functional characteristics. For example, as used within the context of the present invention, the polypeptide fragment has the function of binding to Hsc70/Hsp70, ubiquitinated proteins or valosin containing polypeptide.

As used herein, "Human Fas associated factor 1" protein has an amino acid sequence as follows: 1 - MASNMDREMILADFQACTGIENIDEAITLLEQNNWDLVAAINGVIPQENG ILQSEYGGETIPGPAFNPASHPASAPTSSS SSAFRPVMPS RQIVERQPRM LDFRVEYRDR NVDVVLEDTCTVGEIKQILENELQIPVSKMLLKGWKTGDVEDSTVLKSLHLPKNNSL YVLTPDLPPPSSSSHAGALQESLNQNFMLBTHREVQREYNLNFSGSSTIQEVKRNVY DLTSI PVRHQLWEGWPTSATDDSMCLAESGLSYPCHRLTVGRRSSPAQTREQSEEQITDVH MVSDSDGDDFEDATEFGVDDGEVFGMASSALRKSPMMPENAENEGDALLQFTAEFS SRYGDCHPVFFIGSLEAAFQEAFYVKARDRKLLAIYLHHDESVLTNVFCSQMLCAESI VSYLSQNFITWAWDLTKDSNRARFLTMCNRHFGSVVAQTIRTQKTDQKPLFLIIMGK RSSNEVLNVIQGNTTVDELNIMRLMAAMEIFTAQQQEDIKDGDEREARENVKREQDE AYRLSLEADRAKREAREREMAEQFRLEQIRKEQEEEREAIRLSLEQALPPEPKEENAE PVSKLRIRTPSGEFLERRFLASNKLQIVFDFVASKGFPWDEYKLLSTFPRRDVTQLDPN KSLLEVKLFPQETLFLEAKE - 650 (SEQ ID NO:7). As various amino acid residue numbers are recited in the application with regard to human FAF1 protein, the above sequence is the reference point.

As used herein, "hyperplasia" refers to increased cell production in normal tissue or an organ. Hyperplasia also refers to an excess of normal tissue.

As used herein, "purified" or "isolated" molecule refers to biological molecules that are removed from their natural environment and are isolated or separated and are free from other components with which they are naturally associated.

As used herein, "sample" or "biological sample" is referred to in its broadest sense, and includes any biological sample obtained from an individual, body fluid, cell line, tissue culture, or other source which may contain FAF1, Hsc70/Hsp70, ubiquitinated proteins or valosin containing polypeptide, depending on the type of assay that is to be performed. As indicated, biological samples include body fluids, such as semen, lymph, sera, plasma, urine, synovial fluid, spinal fluid and so on. Methods for obtaining tissue biopsies and body fluids from mammals are well known in the art.

In addition, a "biological sample" obtained from a patient can be referred to either as a "biological sample" or a "patient sample." It will be appreciated that analysis of a "patient sample" need not necessarily require removal of cells or tissue from the patient. For example, appropriately labeled polypeptides (e.g., antibodies or a polypeptide that binds to FAF1 can be injected into a subject and visualized (when bound to the target) using standard imaging technology (e.g., CAT, NMR, EPR, PET and the like). Comparative expression of FAF1 may be examined for cancer diagnostics.

As used herein, "sequence identity", is defined as the percentage of amino acid residues in a candidate sequence that are identical with the amino acid residues in a native polypeptide sequence after aligning the sequences and introducing gaps, if necessary, to achieve the maximum percent sequence identity, and not considering any conservative substitutions as part of the sequence identity. The % sequence identity values are generated by the NCBI BLAST2.0 software as defined by Altschul et al., (1997), "Gapped BLAST and PSI-BLAST: a new generation of protein database search programs", Nucleic Acids Res., 25:3389-3402. The parameters are set to default values, with the exception of the Penalty for mismatch, which is set to -1.

As used herein, the term "specifically binds" refers to a non-random binding reaction between two molecules, for example between an antibody molecule immunoreacting with an antigen, or a non-antibody ligand reacting with another polypeptide.

As used herein, "subject" is a vertebrate, preferably a mammal, more preferably a human.

As used herein, "treatment" is an approach for obtaining beneficial or desired clinical results. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment "Treatment" refers to both therapeutic treatment and prophylactic or preventative measures. Those in need of treatment include those already with the disorder as well as those in which the disorder is to be prevented. "Palliating" a disease means that the extent and/or undesirable clinical manifestations of a disease state are lessened and/or the time course of the progression is slowed or lengthened, as compared to a situation without treatment
As used herein, "vector", "polynucleotide vector", "construct" and "polynucleotide construct" are used interchangeably herein. A polynucleotide vector of this invention may be in any of several forms, including, but not limited to, RNA, DNA, RNA encapsulated in a retroviral coat, DNA encapsulated in an adenovirus coat, DNA packaged in another viral or viral-like form (such as herpes simplex, and adeno-associated virus (AAV)), DNA encapsulated in liposomes, DNA complexed with polylysine, complexed with synthetic polycationic molecules, complexed with compounds such as polyethylene glycol (PEG) to immunologically "mask" the molecule and/or increase half-life, or conjugated to a non-viral protein. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides.

**FAF1 binds to Hsc70/Hsp70**

We examined the cell death inducing property of human Fas associated factor 1 (hFAF1) in heat shock signaling pathway. Employing co-immunoprecipitation and peptide mass fingerprinting using MALDI-TOF MS, we found that hFAF1 binds to 70 kDa heat shock protein family (Hsc70/Hsp70). Interaction mapping indicates that the 82-180 sequence of hFAF1 directly binds to N-terminal region containing the sequence 1-120 of Hsc70/Hsp70. This binding is very tight regardless of ATP and heat shock treatment. Hsc70/Hsp70 and hFAF1 co-localized in cytosol and nucleus, and concentrated to perinuclear region by heat shock treatment. We examined how hFAF1 regulates Hsp70 function and found that hFAF1 inhibited Hsp70 chaperone activity of refolding denatured protein substrates, accelerated heat shock induced SAPK/JNK activation and raised heat shock induced cell death in a binding dependent manner. These results suggest that hFAF1 exhibits cell death inducing property by binding to and inhibiting the chaperone activity of Hsp70, and preventing cells from recovery after stress.

These studies demonstrate interaction between hFAF1 and Hsc70/Hsp70 and the functional significance of this interaction in heat shock induced cell signaling. We previously identified the 1-201 amino acid region of hFAF1 as Fas binding domain using yeast two-hybrid assay (Ryu et al., 1999, Biochem. Biophys. Res. Commun. 262:388-394). The cellular function of hFAF1, a novel protein known to be an adaptor molecule of death receptor Fas, was identified as a member of Fas-death inducing signaling complex (Ryu et al., 2003, J. Biol. Chem. 278:24003-24010). We have shown that the 82-180 amino acid region of hFAF1 directly interacts with N-terminus (1-120) of Hsp70 *in vivo* using 2D-gel electrophoresis and MALDI-TOF MS, gel filtration gel chromatography, and *in vitro* by pull down assay. Hsp homologues such as DnaK and DnaJ were co-purified during the purification of recombinant hFAF1 from *E. coli* lysate.

Hsp70 plays an important protective role in stress-induced cell death. hFAF1 binding can affect this role of Hsp70 in two possible ways. One possibility is that hFAF1 may serve as substrate and become refolded, degraded, or translocated by Hsp70. The second is that hFAF1 may act as modulator of Hsp70 function. Our studies revealed that hFAF1 did not bind to peptide binding domain (PBD), substrate binding sites of Hsp70, but rather bound to N-terminus of ATPase domain of Hsp70, as co-chaperones Bag-1 and Hip binds to ATPase domain. Also the expression level of hFAF1 was not varied in various stresses. This indicates that hFAF1 is not a substrate of Hsp70. We therefore examined whether hFAF1 is involved in the regulation of Hsp70 activity. Transient overexpression of hFAF1 showed inhibition of chaperone activity of Hsp70, while transient expression of N-terminus deleted hFAF1 mutant showed the opposite effect, indicating that hFAF1 may act as a co-chaperone and inhibit the chaperone activity of Hsp70. Recently, co-chaperones of Hsp70 such as Hsp40, Hip, Hop, Bag-1, CHIP, Chap1/PLIC-2 and Chap2Bat3/Scythe have been identified. Hip and Hop facilitates refolding of unfolded proteins and Bag-1, CHIP, and Chap2/Bat3/Scythe attenuated the chaperone activity of Hsp70 (Höhfeld and Jentsch, 1997, EMBO J. 16:6209-6216; Minami et al., 1996, J. Biol. Chem. 271:19617-19624; Schumacher et al., 1994, J. Biol. Chem., 269:9493-9499; Smith et al., 1993, Mol. Cell. Biol. 13:869-876; Takayama et al., 1997, EMBO J. 16:4887-4896; Zeiner et al., 1997, EMBO J. 16:5483-5490; Ballinger et al., 1999, Mol. Cell. Biol. 19:4535-4545; Thress et al., 2001, EMBO J. 20:1033-1041). Since the latter contain ubiquitin like domains, which bind to proteasome, they are thought to be involved in degradation of Hsp70 substrates in proteasome. Bag-1 contains ubiquitin like domain which binds to proteasome, CHIP has U-box domain which has ubiquitin ligase activity, and Chap2/Bat3/Scythe also contains ubiquitin like domain. FAF1 whole sequence is 78-95% identical in vertebrates while 18-21% identical between vertebrates and invertebrates. It contains two ubiquitin related domains, UAS and UBX.

To understand the biological function of hFAF1 reflected by its negative regulation of Hsp70 chaperone activity by hFAF1, we examined the effect of hFAF1 in heat shock responses. Hsp70 seems to play the important role of preventing programmed cell death in response to heat shock, by suppressing the activation of SAPK/JNK (Gabai et al., 1997. J. Biol. Chem. 272:18033-18037; Meriin et al., 1999, Mol. Cell. Biol. 19:2547-2555; Volloch et al., 1999, FEBS Lett. 461:73-76). Using the kinetics of SAPK/JNK activation and deactivation as a marker of cellular sensitivity to stress, we found that the activation of SAPK/JNK in thermotolerant (TT) cells having elevated Hsp70 expression occurred in higher dose of stress and the deactivation in TT cells was much faster than in control (Kim and Lee, 2000, Mol. Cell. Biochem. 229:139-151). Recently we also reported that heat shock induced transient tyrosine phosphorylations of various proteins (Kim et al., 2002, J. Biol. Chem. 277:23193-23207). As shown in Fig. 8, heat shock-induced SAPK/JNK activation in cells overexpressing hFAF1 was accelerated and the deactivation during recovery was dramatically delayed compared to vector transfected control cells. This effect was completely abolished by overexpression of N-terminal truncated hFAF1, lacking affinity for Hsp70 binding. This indicates that overexpression of hFAF1 renders cells more sensitive to heat shock probably due to inhibition of Hsp70 chaperone activity. It is possible that Hsp70 chaperone activity is related to the inhibition of SAPK/JNK activity induced by various stresses and antiapoptotic character. Volloch *et al.,* showed that ABD of Hsp70 is not necessary for the inhibition of SAPK/JNK (Volloch et al., 1998, Cell Stress Chaperones 3:265-271). It seemed that chaperone activity of Hsp70 using ATP hydrolysis as energy source had no more effect on SAPK/JNK activity. However, the role of ABD and chaperone function of Hsp70 is not in total accord (Stege et al., 1994, Exp. Cell Res. 214:279-284). In this study, ABD deletion mutant of Hsp70 showed elevated chaperone activity, although lower than full form of Hsp70, suggesting that ABD which assigned the smaller domain (120-428) in this study than the conventional ABD (1-428) is not indispensable for the chaperone activity. Thus the possibility that chaperone activity of Hsp70 can affect SAPK/JNK activation should not be excluded.

We then examined whether the increased cellular sensitivity to heat shock in hFAF1-overexpressed cell line is related to cell death. HEK293T cells with transient overexpression of Flag-hFAF1 showed lower viability than vector-transfected cells by severe heat shock treatment. Also, Flag-hFAF1ΔV deletion mutant lacking the binding site to Hsp70 showed increased viability than vector transfected cells, suggesting that hFAF1 makes cells more sensitive to heat shock by inhibiting the chaperone activity of Hsp70. The viability of Flag-hFAF1ΔN transfected cells increases possibly because the deletion mutant acts as dominant negative mutant to full form of hFAF1. It is possible that hFAF1 has multiple domains and binding to C-terminal may regulate the ubiquitination pathway.

hFAF1 was originally identified as a fas associated protein. Amino acid sequence 1-201 is binding domain to Fas death domain (Ryu et al., 1999, Biochem. Biophys. Res. Commun. 262:388-394). This region overlaps the Hsp70 interaction region, amino acid residues 82-180. It is possible that hFAF1 binds to fas or Hsp70 depending on various cellular conditions.

hFAF1 is a novel protein, but its cellular function is still unclear. This study suggests that hFAF1 may play a role in stress induced signaling pathway through binding to Hsp70 and inhibiting its chaperone activity. Our demonstration shows that hFAF1 acts as a new co-chaperone of Hsp70 and exerts cell death inducing potential. These functions of hFAF1 may be regulated by post-translational modifications (Jensen et al., 2001, Int. J. Biochem Cell Biol. 33:577-589), and changes in binding at multidomains.

**FAF1 binds to valosin containing protein and multiubiquitinated proteins**

Applicants have demonstrated that hFAF1 binds with valosin-containing protein (VCP) and multiubiquitinated proteins. These interactions appear to play a role in the regulation of proteolytic degradation in the proteasome.

VCP is a member of AAA family (ATPase-associated family with different cellular activities). VCP's yeast homolog, cdc48p, was identified as a cell division cycle protein (24). VCP participates in various cellular processes, including membrane fusion, vesicle-mediated transport and peroxisome assembly (1, 21) through binding to specific cofactors. For example, p47 is a cofactor of VCP in membrane fusion during post-mitotic reassembly of Golgi stack, mediates the binding of VCP to syntaxin 5, analogous to α-SNAP (33). Also Ufd1 and Np14 have been implicated as cofactors of VCP in the proteasome-mediated processing of ER-bound proteins as well as cytosolic proteins (23, 40). Furthermore, cdc48p has been shown to bind to several proteins such as Ufd3p and Ufd2p, implicated in the ubiquitin fusion degradation (UFD) pathway (13, 18). VCP's role in targeting IκBα to the proteasome for degradation (6, 7, 9) has already been noted. These findings suggest that VCP may act as a chaperone that targets many substrates to the proteasome for degradation.

UBA domains have about 45 amino acid residues, preferably about 40 to 50 amino acid residues, and are small (15). They are present in many proteins of the ubiquitination pathway including UBP, E2, E3; UV excision repair proteins such as Rad23 and Dsk2; protein kinases such as p62 involved in cell signaling pathways, and cell cycle control (27, 36). Mueller *et al.* presented the three-dimensional solution structure of UBA domain by NMR spectroscopy (27). Hydrophobic surface patch is a common protein-interacting surface present in diverse UBA domains, and it was suggested that hydrophobic surface patch of UBA domain interacts with the hydrophobic surface on the five-stranded β-sheet of ubiquitin. In fact, it has been reported that Rad23 and Dsk2 containing UBA domains bind to mono-, di-, tetra- and multi-ubiquitin chains. In addition, UBA domain proteins can interact with ubiquitinated target proteins and regulate their proteolysis (4, 12, 37).

The identified N-terminal UBA domain of hFAF1 appears to be important for recruiting and stabilizing the ubiquitinated proteasomal substrates. We found that VCP bound to C-terminal UBX domain of hFAF1 enhances the degradation of ubiquitinated proteins in proteasome and negatively regulated function of hFAF1. Since hFAF1 has many ubiquitin pathway related domains, a relationship between hFAF1 and the ubiquitin pathway was proposed (2). Our studies described here are the first to confirm experimentally that hFAF1 is involved in ubiquitin-proteasome pathway.

hFAF1 interacts with VCP via its C-terminal UBX domain, composed of 80 amino acid residues. The UBX domain reveals a structural relationship with ubiquitin, and has been shown to be present in p47, Y33K, FAF1, UBXD1 and Rep-8 proteins (3). Of these, UBX domain in p47 is the only one whose function is described so far. Apparently, p47 interacts with VCP through its UBX domain (41) and plays a role in membrane fusion processes as an adaptor for VCP (19). It is possible that VCP exerts its various cellular functions including participation in ubiquitin pathway, by interacting with different proteins in a similar fashion.

Our studies also demonstrate that hFAF1 associates with multiubiquitinated proteins of HEK293T cells *in vivo* via its N-terminal UBA domain and that hFAF1 interacts *in vitro* with polyubiquitin chains (Ub₂₋₇) linked by Lys48. As shown in Fig. 15, hFAF1 does not interact with monoubiquitin, but does so strongly with multiubiquitin chains longer than tetraubiquitin chain. This finding is in agreement with previous reports that UBA domain has a higher affinity for tetraubiquitin (Kd, 30 nM) than for monoubiquitin (Kd > 1 mM) (37). Our finding that UBA domain of hFAF1 is aligned with UBA peptide sequence of different proteins, depicted in Fig. 15C, suggest that UBA domain of hFAF1 is not fully conserved with other UBA domains, while the amino acid residues occupying the position on hydrophobic surface patch in three-dimensional structure, which interacts with ubiquitin, are well conserved (Fig. 15C). We conclude that the hFAF1 might represent a new family of proteins with a distinct UBA domain.

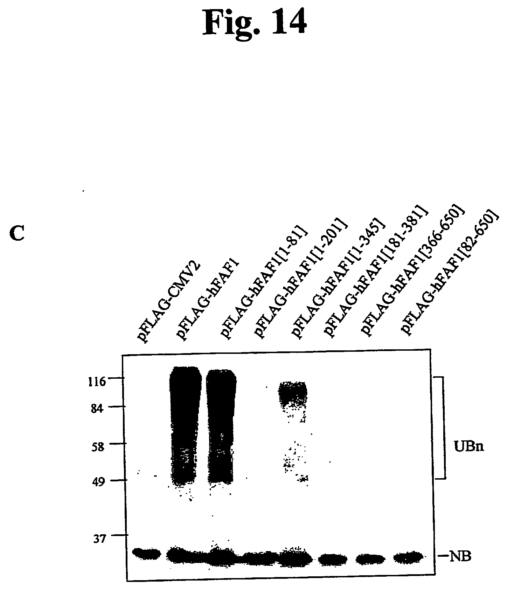

hFAF1 was originally identified as a Fas associated protein, and its amino acid sequence 1-201, was characterized as the region binding to Fas death domain (34). In addition, hFAF1 was shown to interact with Hsp70 via amino acid residues 82-180 (17). We found that Flag-hFAF1[1-201] and Flag-hFAF1[1-345] bound to multiubiquitinated proteins, but these interactions were weaker than those of Flag-hFAF1 full form and Flag-hFAF1[1-81] (Fig. 14). Also, amino acid residues 1-201 and 1-345 contain Fas and Hsp70 interacting domains, in addition to UBA domain. It is possible that structural changes may explain why the truncated construct show weaker interactions, but our results do suggest the possibility that hFAF1 interaction with multiubiquitinated proteins may be regulated by other binding proteins. Further work on the interactions among hFAF1, Fas, Hsp70 and ubiquitinated proteins is clearly required to rule in or out these and other alternate possibilities.

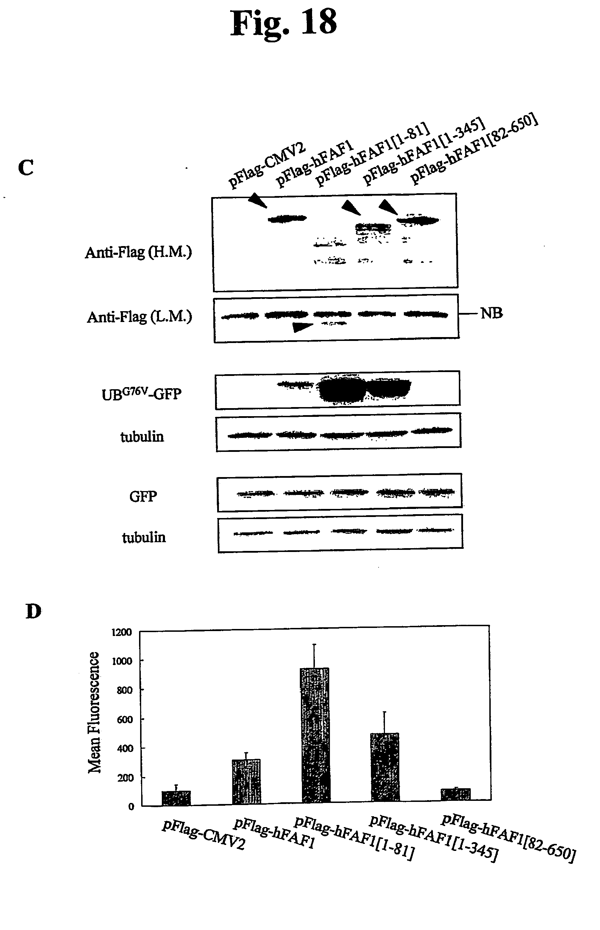

As part of our study of the involvement of hFAF1, interacting with multiubiquitinated proteins, in the degradation of proteasomal substrate, we found that overexpression of full hFAF1 and hFAF1[1-81] interfered with the degradation of artificial substrate, Ub^{G76v}-GFP, and endogenous substrate, IκBα. Constructs not containing UBA domain did not inhibit degradation of Ub^{G76V} -GFP and IκBα. Truncated hFAF1[1-81] and [1-345] inhibited Ub^{G76V}_GFP degradation to a greater degree than full hFAF1 did (Fig. 18), although the binding of full hFAF1 and hFAF1[1-81] to ubiquitinated proteins was similar (Fig. 14). These results suggest that C-terminal UBX domain is required to degrade the ubiquitinated proteins binding to UBA domain.

Our studies using the method developed by *Dantuma et al.* (8, 20) for quantification of ubiquitin-proteasome-dependent proteolysis in living cells, confirmed that hFAF1 inhibited the proteolytic degradation of endogenous as well as artificial substrate (Fig. 19).

Recent studies implicate ubiquitin and ubiquitination in many biological processes, including protein degradation, endocytosis, protein sorting, subnuclear trafficking and gene expression, among others (31). Lys48-linked multiubiquitin chains are the principal proteasomal targeting signal while Lys63-linked chains appear to be non-proteolytic signals (10, 31): Raasi *et al.* using synthetic peptides, found that C-terminal UBA domain of Rad23 binds to Lys48-linked polyubiquitin chains in preference to Lys63/Lys29-linked chains (32). Peng *et al.* reported that Lys6, 11, 27, 33, 48, 63 residues are involved in the ubiquitin chain formation, even though the relative abundance of other Lys-linked chains is less than that of Lys48 and 63-linked chains (30). Our studies showing that N-terminal UBA domain of hFAF1 interacts with both Lys48 and Lys63-linked ubiquitin chains, suggest that hFAF1 may have functions other than proteasomal degradation in ubiquitination pathway. Studies are in progress in our laboratory, designed to identify the ubiquitinated proteins interacting with hFAF1. It is hoped that they will help to explain the cellular function of this interaction.

We found that overexpression of hFAF1 itself causes cell death and that UBA domain is responsible for it Other studies also reveal a link between ubiquitination and apoptosis. Ubiquitinated proteins dramatically increase in several models of apoptosis, triggered variously by DNA-damaging agents, serum deprivation, and γ-irradiation (22, 27). Blockers of protein degradation induce apoptosis in several types of cancer cells. In addition, proteasome inhibitor PS-341, decreased the levels of several anti-apoptotic proteins, such as Bcl-2 and FLIP, and up-regulated proteins implicated in pro-apoptotic cascades, such as Fas, Fas ligand and caspase (25, 38). It seems reasonable to postulate that UBA domain of hFAF1 may function as proteasome inhibitor and regulate the protein levels related to the apoptosis.

**Hyperplasia and Cancer Diagnostic Assay By Detecting FAF1**

The invention also provides diagnostic methods for detecting the presence of FAF1 in a biological sample. This may be assayed either directly (e.g., by assaying polypeptide levels using antibodies elicited in response to FAF1 polypeptide or fragments thereof) or indirectly (e.g., by assaying for antibodies having specificity for FAF1 polypeptide or fragments thereof).

As seen in Figs. 22 and 23, ovarian cancer tissue was compared with normal tissue for FAF1 expression. FAF1 is not expressed in the tumor tissue, but is highly expressed in normal tissue. This indicates that FAF1 is a biological marker for hyperplasia or cancer detection. Although Figs. 22 and 23 show data on ovarian tumor, there is no reason to expect that the inventive cancer diagnostic method should be limited to detecting only ovarian cancer. FAF1 promotes cell death by blocking Hsp70 activity and causing toxicity to a cell by binding to ubiquitinated proteins thereby preventing degradation of these marked proteins. Therefore, the present invention is directed to diagnosing all forms of cancer, including without limitation carcinoma; melanoma and sarcoma. Subtypes of cancer may include without limitation bladder carcinoma, brain tumor, breast cancer, cervical cancer, colorectal cancer, esophageal cancer, endometrial cancer, hepatocellular carcinoma, gastrointestinal stromal tumor (GIST), laryngeal cancer, lung cancer, osteosarcoma, ovarian ancer, pancreatic cancer prostate cancer, renal cell carcinoma or thyroid cancer.

Where diagnosis of a diseased state has already been made, the present invention is useful for monitoring progression or regression of the disease state by measuring the level of expression of FAF1 present in a patient tissue or whereby patients exhibiting attenuated FAF1 production will experience a worse clinical outcome relative to patients producing FAF1 at a higher level.

Presence of the labeled molecule can be detected in the patient using methods known in the art for *in vivo* scanning. These methods depend upon the type of label used. Skilled artisans will be able to determine the appropriate method for detecting a particular label. Methods and devices that may be used in the diagnostic methods of the invention include, but are not limited to, computed tomography (CT), whole body scan such as position emission tomography (PET), magnetic resonance imaging (MRI), and sonography, as well as, electron paramagnetic resonance (EPR) and nuclear magnetic resonance (NMR).

**Labels**

Suitable enzyme labels include, for example, those from the oxidase group, which catalyze the production of hydrogen peroxide by reacting with substrate. Glucose oxidase is particularly preferred as it has good stability and its substrate (glucose) is readily available. Activity of an oxidase label may be assayed by measuring the concentration of hydrogen peroxide formed by the enzyme-labeled antibody/substrate reaction. Besides enzymes, other suitable labels include radioisotopes, such as iodine (¹²⁵I, ¹²¹I), carbon (¹⁴C), sulphur (³⁵S), tritium (³H), indium (¹¹²In), and technetium (^{99m}Tc), and fluorescent labels, such as fluorescein and rhodamine, and biotin.

Examples of suitable radioisotopic labels include ³H, ¹¹¹In, ¹²⁵I, ¹³¹I, ³²p, ³⁵S, ¹⁴C, ⁵¹Cr, ⁵⁷To, ⁵⁸Co, ⁵⁹Fe , ⁷⁵Se, ¹⁵²Eu, ⁹⁰Y, ⁶⁷Cu, ²¹⁷Ci, ²¹¹At, ²¹² Pb, ⁴⁷Sc, ¹⁰⁹Pd, etc. ¹¹¹In is preferred isotope where *in vivo* imaging is used since its avoids the problem of dehalogenation of the ¹²⁵I or ¹³¹I-labeled polypeptide by the liver. In addition, this radionucleotide has a more favorable gamma emission energy for imaging. For example, ¹¹¹In coupled to monoclonal antibodies with 1-(P-isothiocyanatobenzyl)-DPTA has shown little uptake in non-tumors tissues, particularly the liver, and therefore enhances specificity of tumor localization.

Examples of suitable non-radioactive isotopic labels include ¹⁵⁷Gd, ⁵⁵Mn, ¹⁶²Dy, 52^{Tr}, and ⁵⁶Fe. Examples of suitable fluorescent labels include an ¹⁵²Eu label, a fluorescein label, an isothiocyanate label, a rhodamine label, a phycoerythrin label, a phycocyanin label, an allophycocyanin label, an o-phthaldehyde label, and a fluorescamine label. Examples of suitable toxin labels include, Pseudomonas toxin, diphtheria toxin, ricin, and cholera toxin.

Examples of chemiluminescent labels include a luminal label, an isoluminal label, an aromatic acridinium ester label, an imidazole label, an acridinium salt label, an oxalate ester label, a luciferin label, a luciferase label, and an aequorin label. Examples of nuclear magnetic resonance contrasting agents include heavy metal nuclei such as Gd, Mn, and iron. Deuterium may also be used. Other contrasting agents also exist for EPR, PET or other imaging mechanisms, which are known to persons of skill in the art.

Various polypeptides and antibodies may be used to detect FAF1 expression using biochip and biosensor technology. Biochip and biosensors of the present invention may comprise the polypeptides or antibodies, which are specifically recognized by FAF1.

**Cancer or Hyperplasia Treatment By Administering FAF1**

The present invention is also directed to a method of treating hyperplasia or cancer by administering FAF1 polypeptide fragment including FAF1[1-81] that binds to Hcp70/Hsp70 and ubiquitinated protein thereby causing cell death. The type of cancer to be treated is not limited to any particular kind. Types of cancer to be treated may include without limitation carcinoma, melanoma and sarcoma. Subtypes of cancer may include without limitation bladder carcinoma, brain tumor, breast cancer, cervical cancer, colorectal cancer, esophageal cancer, endometrial cancer, hepatocellular carcinoma, gastrointestinal stromal tumor (GIST), laryngeal cancer, lung cancer, osteosarcoma, ovarian ancer, pancreatic cancer prostate cancer, renal cell carcinoma or thyroid cancer.

**Treatment Of Diseases Related To Cell Death By Administering Inhibitors To FAF1**

The present invention can be practiced to treat degenerative diseases caused by cell death such as Alzheimer's disease by preventing the activity of FAF1. Such inhibitor may be chemical, polypeptide or nucleic acid based. For example, RNAi made against FAF1 may be administered to a subject to inhibit FAF1 activity and prevent cell death.

**Gene Therapy**

In a specific embodiment, nucleic acids comprising sequences encoding the FAF1 polypeptide or fragment are administered to treat, inhibit or prevent a disease or disorder associated with cancer, by way of gene therapy. Gene therapy refers to therapy performed by the administration to a subject of an expressed or expressible nucleic acid. In this embodiment of the invention, the nucleic acids produce their encoded protein that mediates a therapeutic effect.

Any of the methods for gene therapy available in the art can be used according to the present invention.

In a preferred aspect, nucleic acid sequences may encode a FAF1 polypeptide or fragment, in which the nucleic acid sequences are part of expression vectors that express the polypeptides in a suitable host In particular, such nucleic acid sequences have promoters operably linked to the polypeptide coding region, said promoter being inducible or constitutive, and, optionally, tissue-specific. In another particular embodiment, nucleic acid molecules are used in which the polypeptide coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA 86:8932-8935 (1989); Zijlstra et al., Nature 342:435-438 (1989).

Delivery of the nucleic acids into a patient may be either direct, in which case the patient is directly exposed to the nucleic acid or nucleic acid- carrying vectors, or indirect, in which case, cells are first transformed with the nucleic acids *in vitro,* then transplanted into the patient These two approaches are known, respectively, as *in vivo* or *ex vivo* gene therapy.

In a specific embodiment, the nucleic acid sequences are directly administered *in vivo*, where it is expressed to produce the encoded product This can be accomplished by any of numerous methods known in the art, e.g., by constructing them as part of an appropriate nucleic acid expression vector and administering it so that they become intracellular, e.g., by infection using defective or attenuated retrovirals or other viral vectors, or by direct injection of naked DNA, or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem. 262:4429-4432 (1987)) (which can be used to target cell types specifically expressing the receptors) and so on.

In a specific embodiment, viral vectors that contain nucleic acid sequences encoding the polypeptide are used. The nucleic acid sequences encoding the polypeptide to be used in gene therapy are cloned into one or more vectors, which facilitates delivery of the gene into a patient. Retroviral vectors, adenoviral vectors and adeno-associated viruses are examples of viral vectors that may be used. Retroviral vectors contain the components necessary for the correct packaging of the viral genome and integration into the host cell DNA.

Adenoviruses are especially attractive vehicles for delivering genes to respiratory epithelia because they naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. In addition, adeno-associated virus (AAV) has also been proposed for use in gene therapy.

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to, isolate those cells that have taken up and are expressing the transferred gene. Those cells are then delivered to a patient

**Therapeutic Composition**

In one embodiment, the present invention relates to treatment for cancer that are characterized by lack of expression of FAF1. In this way, the inventive therapeutic compound may be administered to human patients who are either suffering from, or prone to suffer from the disease by providing compounds that inhibit the Hsp70 chaperoning activity or ubiquitinated protein degradation.

In another embodiment, the present invention relates to treating various diseases that are characterized by excessive cell growth, which include but are not limited to various types of cancer.

The formulation of therapeutic compounds is generally known in the art and reference can conveniently be made to Remington's Pharmaceutical Sciences, 17th ed., Mack Publishing Co., Easton, Pa., USA. For example, from about 0.05 µg to about 20 mg per kilogram of body weight per day may be administered. Dosage regime may be adjusted to provide the optimum therapeutic response. For example, several divided doses may be administered daily or the dose may be proportionally reduced as indicated by the exigencies of the therapeutic situation. The active compound may be administered in a convenient manner such as by the oral, intravenous (where water soluble), intramuscular, subcutaneous, intra nasal, intradermal or suppository routes or implanting (eg using slow release molecules by the intraperitoneal route or by using cells e.g. monocytes or dendrite cells sensitised *in vitro* and adoptively transferred to the recipient). Depending on the route of administration, the peptide may be required to be coated in a material to protect it from the action of enzymes, acids and other natural conditions which may inactivate said ingredients.

For example, the low lipophilicity of the peptides will allow them to be destroyed in the gastrointestinal tract by enzymes capable of cleaving peptide bonds and in the stomach by acid hydrolysis. In order to administer peptides by other than parenteral administration, they will be coated by, or administered with, a material to prevent its inactivation. For example, peptides may be administered in an adjuvant, co-administered with enzyme inhibitors or in liposomes. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEP) and trasylol. Liposomes include water-in-oil-in-water CGF emulsions as well as conventional liposomes.

The active compounds may also be administered parenterally or intraperitoneally. Dispersions can also be prepared in glycerol liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The prevention of the action of microorganisms can be brought about by various antibacterial and antifungal agents, for example, chlorobutanol, phenol, sorbic acid, theomersal and the like. In many cases, it will be preferable to include isotonic agents, for example, sugars or sodium chloride. Prolonged absorption of the injectable compositions can be brought about by the use in the composition of agents delaying absorption, for example, aluminium monostearate and gelatin.

Sterile injectable solutions are prepared by incorporating the active compounds in the required amount in the appropriate solvent with various other ingredients enumerated above, as required, followed by filtered sterilization. Generally, dispersions are prepared by incorporating the various sterile active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof

When the peptides are suitably protected as described above, the active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions is such that a suitable dosage will be obtained. Preferred compositions or preparations according to the present invention are prepared so that an oral dosage unit form contains between about 0.1 µg and 2000 mg of active compound.

The tablets, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

As used herein "pharmaceutically acceptable carrier and/or diluent" includes any and all solvents, dispersion media, coatings antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired.

The principal active ingredient is compounded for convenient and effective administration in effective amounts with a suitable pharmaceutically acceptable carrier in dosage unit form. A unit dosage form can, for example, contain the principal active compound in amounts ranging from 0.5 µg to about 2000 mg. Expressed in proportions, the active compound is generally present in from about 0.5 µg/ml of carrier. In the case of compositions containing supplementary active ingredients, the dosages are determined by reference to the usual dose and manner of administration of the said ingredients.

Delivery Systems

Various delivery systems are known and can be used to administer a compound of the invention, e.g., encapsulation in liposomes, microparticles, microcapsules, recombinant cells capable of expressing the compound, receptor-mediated endocytosis, construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody or a peptide of the invention, care must be taken to use materials to which the protein does not absorb. In another embodiment, the compound or composition can be delivered in a vesicle, in particular a liposome. In yet another embodiment, the compound or composition can be delivered in a controlled release system. In one embodiment, a pump may be used. In another embodiment, polymeric materials can be used. In yet another embodiment, a controlled release system can be placed in proximity of the therapeutic target, i.e., the brain, thus requiring only a fraction of the systemic dose.
A composition is said to be "pharmacologically or physiologically acceptable" if its administration can be tolerated by a recipient animal and is otherwise suitable for administration to that anima Such an agent is said to be administered in a "therapeutically effective amount" if the amount administered is physiologically significant An agent is physiologically significant if its presence results in a detectable change in the physiology of a recipient patient.

The present invention is not to be limited in scope by the specific embodiments described herein. Indeed, various modifications of the invention in addition to those described herein will become apparent to those skilled in the art from the foregoing description and accompanying figures. Such modifications are intended to fall within, the scope of the appended claims. The following examples are offered by way of illustration of the present invention, and not by way of limitation.

### EXAMPLE

**EXAMPLE 1 -** EXPERIMENTAL PROCEDURES RELATED TO FAF1 BINDING TO HCP70/HSP70

Example 1.1 - Plasmids

Full length human hsp70 was subcloned into the pGEX-4T-1 vector. pGEX-4T-1/Hsp70ΔABD (1-119, 429-640), The constructs pGEX-4T-1/Hsp70N (1-119) was prepared by PCR using sense primer 5'-GAAGAATTCATGGCCAAAGCCGCGGCAG-3' (SEQ ID NO:1) and anti-sense primer 5'-GCATCTCGAGCGAGATCTCCTCGGGGTA-3' (SEQ ID NO:2) and subcloned into the pGEX-4T-1 vector. pGEX-4T-1/Hsp70ΔN (120-640) was prepared by PCR using sense primer 5'-CGAGGAATTCATGTCCATGGTGCTGACC-3' (SEQ ID NO:3) anti-sense primer 5'-GGCCTCGAGCTAATCTACCTCCTCAATG-3' (SEQ ID NO:4) and subcloned into the pGEX-4T-1 vector. pGEX-4T-1/Hsp70ΔPBD (1-435, 619-640) were prepared as described previously (Park et al., 2001, EMBO J. 20:446-456). The constructs pFlag-CMV-2/hFAF1, pFLAG-CMV-2/hFAF1 [1-201], pFLAG-CMV-2/hFAF1 [1-345], pFLAG-CMV-2/hFAF1 [82-650], pFLAG-CMV-2/hFAF1 [366-650] (hFAF1ΔN), pFLAG-CMV-2/hFAF1 [181-381], and pCDNA/hFAF1 were prepared described previously (Hartl, 1996, Nature 38:571-580). pCytluc (pRSVTLL/V) encodes cytoplasmic luciferase was provided by S. Subramani (University of California, San Diego).

Example 1.2 - Cells and Reagents

Human embryonic kidney epithelial (HEK293T) cells were grown and maintained in high glucose Dulbecco's modified Eagle's medium (DMEM) supplemented with 10% fetal bovine serum at 37°C and 5% CO₂. Monoclonal anti-Flag antibody M2 was purchased from Sigma. Monoclonal anti-Hsc70/Hsp70 antibody was obtained from StressGen. Polyclonal anti-hFAF1 antibody was generated in our group.

Example 1.3 - Transient Transfection

HEK293T cells were transfected with expression plasmid using the calcium phosphate precipitation method. Cells were seeded in 10 cm plates a day before transfection at the density of 1.5 X 10⁶ cells and transiently transfected with 6-12 µg of expression plasmid by calcium phosphate method. The fresh medium was changed at 6 h after transfection and cultured for additional 18 h.

Example 1.4- Metabolic Labeling and Immunoprecipitation

Cells were metabolically labeled with 2 µCi/ml [³⁵S]methionine in methionine half-free medium for 18 h. Cells were disrupted with a buffer containing protease inhibitors (50 mM Tris, pH 8.0, 150 mM NaCl, 2 mM EDTA, 0.5% NP-40, 1 mM PMSF, 0.5 mM DTT, 5 µg/ml aprotinin, 1 µg/ml leupeptin, 5 mM Na₃VO₄, 5 mM NaF) for 30 min on ice. The lysates were centrifuged at 12,000 rpm for 1 h and the supernatant was incubated for 3 h at 4°C with monoclonal anti-Flag M2-affinity crosslinking agarose beads or incubated for 2 h with anti-Flag antibody and for additional 1 h with protein A beads at 4°C. The beads were washed more than three times with 1 ml of lysis buffer.

Example 1.5 - Two-dimensional Gel Electrophoresis

The protein samples were mixed with a buffer containing 9.5 M urea, 2% Triton X-100, 5% β-mercaptoethanol, 1 mM PMSF, 5 µg/ml aprotinin, 10 µg/ml pepstatin A, 10 µg/ml leupeptin, 1 mM EDTA, 10 mM Na₃VO₄,10 mM NaF for 30 min at room temperature and electrofocused in 7 cm Immobiline DryStrips (pH 4-7) with the Amersham IPGphor. The following focusing protocol was used: 50 µA per strip at 20°C; (1) rehydration for 16 h; (2) 500 V for 1 h (step and hold); (3) 1000 V for 1 h (step and hold); (4) 8000 V for 3 h (step and hold). After electrofocusing, the strips were shaken for 15 min with equilibration buffer (1.5 M Tris-Cl, pH 8.8, 6 M urea, 30% glycerol, 2% SDS, 10 mg/ml DTT) and loaded onto Bio-Rad mini 2D-gel SDS-PAGE.

Example 1.6 - *In vitro* binding assay

GST-Hsp70 deletion mutant expressed *E. coli* cytosol was incubated with glutathione sepharose 4B beads in PBS containing 0.1% Triton X-100 for 3 h at 4°C. The beads were washed three times with PBS containing 0.1% Triton X-100. Binding assays were performed with Hsp70 deletion mutants bound to glutathione-sepharose 4B beads and hFAF1 cleaved by thrombin from GST-hFAF1 in binding buffer (50 mM NaCl with 1 mg of bovine serum albumin per ml) with rocking for 3 h at 4°C. The beads were washed five times with PBS containing 0.5% NP-40, resuspended in 2X gel sample buffer, resolved with SDS-PAGE, and coomassie blue stained. After obtaining the gel image, the proteins in the gel were transferred to PVDF membrane and immunostained with polyclonal anti-hFAF1 antibody.

Example 1.7 - Confocal microcopy

For morphological studies, cells were grown on coated coverslips, transiently transfected, and treated with heat shock. The cells were gently rinsed in HBSS, and fixed with 4% paraformaldehyde in HBSS for 10 min at room temperature. After washing with HBSS, the cells were permeabilized by incubating with 0.1% Triton X-100 in HBSS for 10 min at room temperature before immunostaining. Nonspecific protein absorption was inhibited by incubation of the cells for 1 h in HBSS containing 3% BSA, 0.2% Tween 20, and 0.2% gelatin. For Hsc70/Hsp70 staining, the cells were incubated with mouse monoclonal Hsc7O/Hsp70 antibody (StressGen) diluted at 1:150 in HBSS containing 1% sucrose and 1% BSA for 2 h at 37°C. After washing three times of washes with HBSS, the cells were incubated for 1 h with Texas red-conjugated rabbit anti-mouse (Molecular Probes) diluted at 1:200 and subsequently washed with HBSS. Flag-hFAF1 was stained with FITC-labeled monoclonal M2 anti-Flag antibody diluted at 1:200 for 1 h at room temperature. Confocal microscopy was performed with a Multi-photon Radiance 2000 (Bio-Rad) connected to a microscope (Nikon Eclipse TE 300).

Example 1.8 - In-Gel Digestion and Mass spectrometric analysis

The cellular proteins were separated on 2D-gel electrophoresis and stained with coomassie blue or silver. The gel spots were excised with a scalpel, crushed and destained by washing with 25 mM NH₄HCO₃/50% acetonitrile. The silver stained gel was destained by washing with 15 mM K₄FeCN₆/50 mM sodium thiosulfate. After crushing, the gels were dehydrated by addition of acetonitrile, rehydrated by adding 10-20 µl of 25 mM ammonium bicarbonate with 10 ng/µl of sequencing grade trypsin (Promega), and incubated at 37°C for 12-15 h. Peptides were extracted by adding 30 µl of solution containing 60% acetonitrile/0.1% trifluoroacetic acid. The extraction was repeated three times and completed by adding 20 µl of acetonitrile. The extracted solutions were pooled and evaporated to dryness in a SpeedVac vacuum centrifuge. Samples were reconstituted in 10 µl of 0.1% TFA and treated with ZipTips containing C18 resin (Millipore) according to the manufacturer's instructions. The washed peptides were eluted with saturated matrix solution (α-cyano-4-hydroxycinnamic acid in 60% acetonitrile/0.1% trifluoroacetic acid). Peptide mixtures were analyzed with MALDI-TOF MS using a delayed ion extraction and ion mirror reflector mass spectrometer (Voyager-DE STR; Applied Biosystems, Inc.). External calibration was carried out using Sequazyme Peptide Mass Standard Kit (PerSeptive Biosystems) and internal calibration, by using the autolytic peaks of trypsin. This procedure typically results in mass accuracies of 50 ppm. For interpretation of the mass spectra, we used the Ms-Fit program available on the World Wide Web site of the University of California at San Francisco.

Example 1.9 - Luciferase reactivation assay

Cells were transiently transfected with pCytluc and Flag-tagged Hsp70 or hFAF1. Twenty-four hours after transfection, the cells were transferred into tissue culture dishes in medium with 20 µg/ml cycloheximide for 30 min at 37°C. Luciferase was inactivated by heating the cells at 45°C for 15 min and recovered at 37°C for various times. Luciferase activities of the harvested cells were measured using Luciferase assay kit (Promega).

Example 1.10 - SAPK/JNK assay

SAPK/JNK was assayed using glutathione-S-transferase-c-Jun 1-89 peptide (provided by Dr. J. R. Woodgett) as a substrate after immunoprecipitation with anti-JNK antibody (Santa Cruz Biotechnology). Cell pellets were homogenized with a buffer A containing 20 mM HEPES, pH 7.4, 50 mM β-glycerophosphate, 2 mM EGTA, 1 mM dithiothreitol, 1 mM Na₃VO₄, 1% Triton X-100, 10% glycerol, 2 µM leupeptin, 400µM phenylmethylsulfonyl fluoride, and 10 units/ml aprotinin. The supernatant containing 1 mg of protein was incubated with antibodies against mammalian SAPK/JNK and then with protein A-Sepharose 4B suspension. The immunocomplexes were washed three times and then incubated with a kinase buffer (50 mM Tris-Cl, pH 7.4, 10 mM MgCl₂, 2 mM EGTA, 1 mM DTT, 0.1% Triton X-100) supplemented with glutathione S-transferase (GST)-c-Jun and [γ-³²P]ATP, for 20 min at 37°C. To stop the reaction, 2x gel sample buffer was added. Samples were subjected to 12% SDS-PAGE and the gels were stained, destained and dried. Phosphorylation of c-Jun was measured by BAS2500 (Fuji).

**EXAMPLE 2** - RESULTS

Example 2.1- Identification of Hsc70 and Hsp70 as hFAF1 interacting protein *in vivo*

To identify the proteins interacting with hFAF1, we performed coimmunoprecipitation experiments. Flag-tagged hFAF1 was transiently overexpressed in HEK293T cells and cellular proteins were metabolically labeled with 2 µCi/ml [³⁵S]methionine. hFAF1-interacting proteins were divided into two fractions and analyzed by 2D-gel electrophoresis. One fraction was transferred to NC membrane for autoradiogram and Western blot analysis and the other was silver stained for visualization (Fig. 1). Protein spots detected in the autoradiogram were cut out from the corresponding silver stained gel and subjected to in-gel digestion with trypsin and mass peptide fingerprint analyses using MALDI-TOF MS. We identified spot number 1 and 2 as heat shock protein 70 cognate (Hsc70) and heat shock protein 70 inducible (Hsp70), respectively (Table 1). Binding to Hsc70 and Hsp70 were reconfirmed by western blot analysis of the NC membrane with monoclonal anti-Hsc70/Hsp70 antibody (Fig. 1C). To further confirm the binding, cell lysate from Flag-hFAF1 overexpressed cell line was separated on gel filtration column chromatography, and binding complex of hFAF1 and Hsc70/Hsp70 was coeluted at 250-300 kDa fraction (data not shown).

**Table 1. Flag-hFAF1 binding proteins identified by 2D-gel and MALDI-TOF**

| Spot No. | Identified protein | NCBI GI No. | Mass (Da) | pI | Coverage (%) |
|---|---|---|---|---|---|
| 1 | Hsc70 | 13273304 | 70899 | 5.4 | 29 |
| 2 | Hsp70 | 386785 | 69869 | 5.4 | 30 |

In order to see if endogenous hFAF1 interacts with endogenous Hsc70/Hsp70 in *vivo,* we performed immunoprecipitation studies with polyclonal anti-hFAF1 antibody in HEK293T cells. Fig. 2 shows that endogenous Hsc70/Hsp70 exist in the hFAF1 protein complex *in vivo.* In addition, the recombinant hFAF1 expressed in *E. coli* can bind to bacterial Hsp70, DnaK (data not shown), suggesting that hFAF1 has intrinsic binding perperty to Hsp70.

Example 2.2 - Hsc70/Hsp70 interact with the amino acid 82-180 of hFAF1 *in vivo*

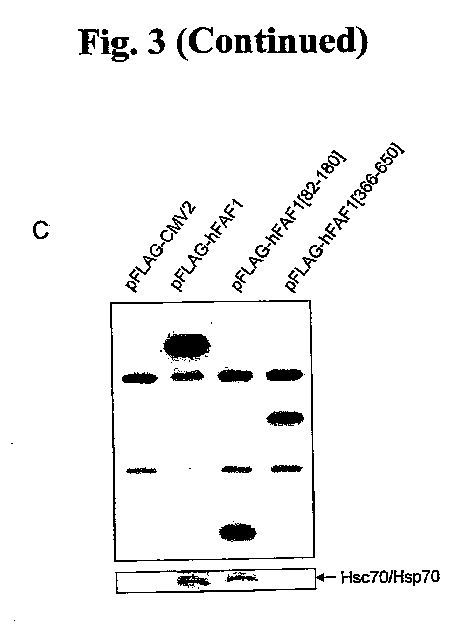

To characterize the hFAF1 domains necessary for interaction with Hsp70, we examined binding between endogenous Hsc70/Hsp70 and a series of transiently transfected Flag-hFAF1 deletion mutants (Fig. 3A). HEK293T cells were transfected with various Flag-tagged hFAF1 deletion mutants and immunoprecipitated by anti-Flag M2-agarose crosslinking affinity gel. The precipitates were analyzed by western blot analysis using anti-Flag antibody (Fig. 3B upper panel) and anti-Hsc70/Hsp70 antibody (Fig. 3B lower panel). Hsc70/Hsp70 did not bind to anti-Flag M2-agarose crosslinking affinity gel of vector-transfected cells, bound only negligibly to amino acid sequences of 366-650 and 181-381 of flag-tagged hFAF1, but bound well to N-terminal hFAF1 fragments containing amino acid sequences, 1-201, 1-345, and 82-650. These data showed that amino acid sequence, 82-180 of hFAF is necessary for binding with Hsc70/Hsp70. It was confirmed in Fig. 3C. The amino acid 82-180 of hFAF1 bound to endogenous Hsc70/Hsp70. This region includes the Fas death domain-binding domain (amino acid residues 1-201) (Ryu et al., 1999, Biochem. Biophys. Res. Commum 262:388-394).

Example 2.3 - hFAF1 binds directly to the N-terminal domain of Hsp70 *in vitro*

To examine whether hFAF1 might be associated directly or indirectly with Hsp70, in an accessory protein-mediated processes, we carried out *in vitro* pull down assay. We prepared various truncated forms of GST-Hsp70 (Fig. 4A): full Hsp70, 1-640; 1-120; 120-640; 1-119 and 428-640; and 1-435 and 616-640. Various recombinant GST-Hsp70s were immobilized to glutathione-sepharose beads, and incubated with purified hFAF1 in the presence of 10 mM ATP. We found direct interaction between hFAF1 and GST-Hsp70 (1-120) by immunoblotting with anti-hFAF1 antibody (Fig. 4B). In the absence of ATP, the binding between Hsp70 and hFAF1 was not affected (Fig. 4C). These data suggest that hFAF1 directly interacts with N-terminus of Hsp70 and that ATP-induced conformational changes of Hsp70 did not cause any changes in binding.

Example 2.4 - Heat shock does not affect the interaction between Hsp70 and hFAF1

We examined the effect of heat shock on the interaction between hFAF1 and Hsc70/Hsp70 *in vivo.* HEK293T cells transiently transfected with pFlag-CMV-2 vector or Flag-tagged hFAF1 were heat shocked at 45°C for 45 min and recovered for various times at 37°C, and immunoprecipitated with anti-Flag M2-agarose crosslinking affinity gel. Immunoprecipitates were analyzed by western blot analysis using anti-Hsc70/Hsp70 (Fig. 5 upper panel) and anti-Flag M2 antibody (Fig. 5 lower panel). When we quantitatively analyzed the ratio of Hsc70/Hsp70 to Flag-hFAF1 in a linear range using LAS1000 (Fuji), Hsc70 interactions did not change during recovery after heat shock. This suggests that the interaction between hFAF1 and Hsc70/Hsp70 is homeostatic and is not affected by heat shock treatment.

Example 2.5 - Subcellular localization of hFAF1 and Hsc70/Hsp70 were changed by heat shock

Since the interaction between hFAF1 and Hsc70/Hsp70 was unaffected by ATP and stress, the question arises whether the localization of binding complex is specific and is changed by heat shock. Flag-hFAF 1 transiently overexpressed in Hela cells were treated with heat shock at 45°C for 30 min and recovered at 37°C after 24 h. Hsp70/Hsc70 proteins were stained with monoclonal anti-Hsc70/Hsp70 antibody and secondary antibody labeled with Texas red and Flag-hFAF1 with FITC-labeled anti-Flag M2 monoclonal antibody. hFAF1 and Hsp70 normally appeared in the cytosol and nucleus (Figs. 6A and B. When two images were merged, cytosolic and nuclear region showed bright yellow color, suggesting co-localization of two proteins (Fig. 6C). When the cells were treated with heat shock at 45°C for 30 min, immediately after heat shock, hFAF1 and Hsp70 colocalized in perinuclear region and speckles as bright yellow in cytosol (Fig. 6F). After 24 h recovery at 37°C, hFAF1 relocalized mainly to the nucleus and cytosol and Hsp70 showed enrichment in nucleus when compared to the control cells (Fig. 6G and H. Again the two proteins co-localized in perinuclear region (Fig. 6I). These results show that the sites of co-localization of hFAF1 and Hsp70 vary between cytosolic and nuclear region and perinuclear region, depending on heat shock and recovery conditions.

Example 2.6 - HFAF1 inhibits Hsp70-mediated reactivation of heat-denatured firefly *luciferase in vivo*

Previous reports showed the activity of transiently expressed luciferase in cells can serve as a marker of chaperone activity of Hsp70 *in vivo* (Michels et al., 1995, Eur. J. Biochem. 234:382-389; Nollen et al., 2000, Mol. Cell. Biol. 20:1083-1088). To examine whether Flag-tagged Hsp70 accelerates the reactivation of heat-denatured luciferase *in vivo,* we transiently transfected Flag-tagged Hsp70 or Hsp70 ATP binding domain deletion mutant (Hsp700ABD, 1-119 and 429-640) in HEK293T cells. We co-expressed firefly luciferase, whose enzymatic activity during recovery from thermal inactivation is highly dependent on chaperone activity. The overexpression of each protein was confirmed by western blot analysis using anti Hsp70 antibody (Fig. 7A) because we had problem detecting Flag-Hsp70 with anti-Flag antibody. The cells were heat shocked at 45°C for 15 min and recovered for indicated times at 37°C, harvested and lysed, and luciferase assay performed (Fig. 7B). Overexpression of Hsp70 alone is sufficient to enhance reactivation of luciferase activity during recovery at 37°C. However, in Hsp700ΔBD transfected cells, the reactivation of luciferase was slower than in Hsp70 expressed cells but faster than in control cells, suggesting that heat shock inactivated luciferase was reactivated by the chaperone activity of Hsp70, and that ATP binding domain is not essential for chaperone activity *in vivo.*

To examine the effect of hFAF1 on the chaperone activity of Hsp70, HEK293T cells were transfected with Hsp70 with or without Flag-tagged hFAF1. The overexpression of Flag-tagged hFAF1 was confirmed by western blot analysis using anti-Flag and anti-hFAF1 antibody (Fig. 7C). As Hsp70 nearly co-migrates with hFAF1, we did not show the Hsp70 in Fig. 7C panel. Co-transfection of Flag-hFAF1 with Hsp70 significantly reduced the renaturation activity of Hsp70 (Fig. 7D).

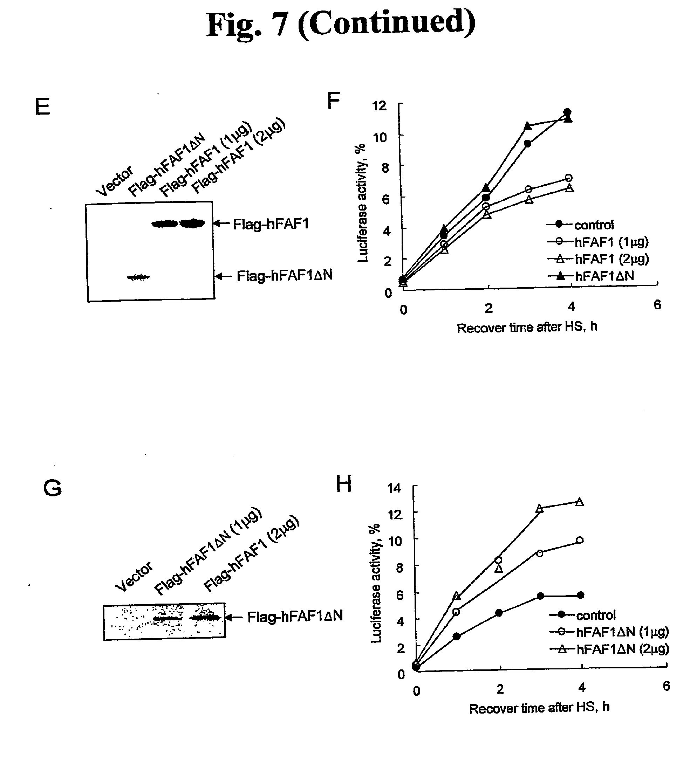

To confirm that the inhibitory effect of hFAF1 is through binding to endogenous Hsp70, HEK293T cells were transfected with Flag-tagged hFAF1 or a deletion mutant, amino acid sequence 366-650 of Flag-hFAF1 (Flag-hFAF1ΔN), lacking the N-terminal domain required for Hsp70 interaction without Hsp70 overexpression. Expression of Flag-hFAF and Flag-hFAF1ΔN was monitored by western blot analysis (Fig. 7E). Transfection of Flag-CMV-2 vector plasmid alone caused basal level reactivation of heat-denatured cytoplasmic luciferase up to 11% after 4h at 37°C, whereas in the presence of Flag-hFAF1, luciferase activity was significantly reduced to 6 or 7%. However, expression of Flag-hFAF1ΔN did not inhibit Hsp70 chaperone activity (Fig. 7F). To confirm the dominant negative like effect of the overexpression of Flag-hFAF1ΔN hFAF1 lacking the N-terminal domain required for Hsp70 interaction inf Fig. 7F, we transfected 1 µg and 2 µg of Flag-hFAF1ΔN vector DNA in HEK293T cells. Expression of Flag-hFAF1ΔN was monitored by western blot analysis (Fig. 7G). Transfection of Flag-hFAF1ΔN increased the renaturation activity of Hsp70 in dose-dependent manner (Fig. 7H). These studies establish that hFAF1 acts as an inhibitor of the chaperone activity of Hsp70, and that the N-terminal residues of hFAF1 play a role in this inhibition of Hsp70 chaperone activity.

Example 2.7 - Overexpression of hFAF1 increases cell sensitivity to heat shock stress

Activation of SAPK/JNK is required for stress induced apoptosis and Hsp70 overexpression prevents apoptosis by suppressing the activation of SAPK/JNK (Gabai et al., 1997. J. Biol. Chem. 272:18033-18037; Volloch et al., 1998, Cell Stress Chaperones 3:265-271; Meriin et al., 1999, Mol. Cell. Biol 19:2547-2555). We have shown that cells being tolerant to stress by expressing the elevated Hsp70 were less sensitive to stress, activated the SAPK/JNK in response to higher dose of stress, and had faster deactivation rate of SAPK/JNK in same dose of stress. It would thus appear that kinetics of SAPK/JNK activation and deactivation can serve as a marker of cellular sensitivity to various stresses (Kim and Lee, 2000, Mol. Cell. Biochem 229:139-151).

We then examined the effect of overexpression of hFAF1 on heat shock-induced cellular response, using SAPK/JNK activation as a marker. We transfected HEK293T cells with Flag-CMV-2 vector, Flag-tagged hFAF1, or Flag-tagged hFAF1ΔN. Transfected HEK293T cells were treated with heat shock at 45°C for 45 min and SAPK/JNK activities were measured (Fig. 8). The kinetics of SAPK/JNK activation in both control and Flag-hFAF1ΔN expressing cells, were similar: there was 13 fold activation immediately after heat shock and deactivation to the basal level after 7 h recovery. On the other hand, SAPK/JNK activity in Flag-hFAF1 expressing cells increased 16 fold activation immediately after heat shock and lasted up to 2 h: deactivation was significantly delayed up to 7 h. This indicates that hFAF1-expressing cells were more sensitive to heat shock and less able to recover. Also the hyper-activation of SAPK/JNK observed in heat-shocked Flag-hFAF1 expressing cells was absent in heat-shocked Flag-hFAF1ΔN expressing cells. This suggests that overexpression of hFAF1 renders cells more sensitive to heat shock and more prone to apoptosis.

Example 2.8 - hFAF1 overexpression increases heat shock induced cell death

To confirm the effect of hFAF1 on stress-induced cell death, we examine the cell viability after heat shock. HEK293T cells transfected with Flag-CMV-2 vector, Flag-tagged hFAF1, or Flag-tagged hFAF1ΔN were treated with heat shock at 45°C for 90 min. After recovery at 37°C for 48 h, the percentages of viable cells were determined by trypan blue dye exclusion assay. Flag-hFAF1 overexpressed HEK293T cells displayed reduced survival compared with vector transfectants in contrast to Flag-hFAF1AN transfected cells which showed enhanced survival (Fig. 9), confirming that complexing with hFAF1 decreased chaperone activity of Hsp70, and made cells more sensitive to stress. Flag-hFAF1ΔN expressing cells, which lack of Hsp70 binding affinity, showed tolerance to heat shock. This may be due to increased chaperone activity of Hsp70 or some unknown role of hFAF1 C-terminus.

Example 2.9 - FAF1 sequences are well conserved in vertebrates

To determine whether FAF1 is well conserved through evolution, we aligned long and short form of hFAF1s (Ryu et al., 1999, Biochem. Biophys. Res. Commun. 262:388-394), rat, mouse, quail, *Danio rerio, Caenorhapditis elegans,* and fly FAF1. As shown in Fig. 10, FAF1 whole sequence is well conserved as well as 82-180 amino acid region to which Hsp70 binds. It is 78-95% identical in vertebrates while 18-21% identical between vertebrates and invertebrates. This suggests that FAF1 plays an essential role throughout the vertebrates.

**EXAMPLE 3** - EXPERIMENTAL PROCEDURES RELATED TO FAF1 BINDING TO VCP AND UBIQUINATED PROTEINS

Example 3.1 - Cell Culture, cDNA Expression and Antibodies

The 293 human embryonic kidney epithelial (HEK293T) cells were grown and maintained in high glucose Dulbecco's modified Eagle's medium (DMEM) and Jurkat T lymphoma cells (E6-1 clone) were grown in RPMI-1640 supplemented with 10% fetal bovine serum (FBS) at 37°C and 5% CO₂.

For expression in mammalian cells, the constructs encoding pFlag-CMV-2-hFAF1 full, pFlag-CMV-2-FAF1 [1-81], pFlag-CMV-2-FAF1 [1-201], pFlag-CMV-2-FAF1 [1-345], pFlag-CMV-2-FAF1 [181-381], pFlag-CMV-2-FAF1 [366-650], pFlag-CMV-2-FAF1 [567-650], pFlag-CMV-2-FAF1 [82-650] and GST-hFAF1 were prepared as previously described (35). Flag-VCP and GST-VCP were cloned into the EcoRI and Sail sites of the pFlag-CMV-2 vector and pGEX-4T-1 vector using VCP cDNA clone obtained from human cDNA library. Ub^{G76V}-GFP vector cloned in the EcoRI and BamHI sites of the EGFP-N1 vector was provided by N.P. Dantuma (Karolinska Institutet, Sweden) (8, 20). Monoclonal anti-Flag antibody (M2), monoclonal anti-Flag M2 agarose crosslinking affinity gel and MTT assay kit were purchased from Sigma (St Louis, MO, USA) and polyubiquitin chains (Ub₂₋₇) from Affiniti Research Products (BIOMOL International LP, UK). Monoclonal anti-tubulin, anti-GFP and anti-IκBα antibodies were purchased from Santa Cruz Biotechnology (Santa Cruz, CA, USA), monoclonal anti-ubiquitin antibody from Chemicon (Temecula, CA, USA), monoclonal anti-VCP antibody from Research Diagnostics (Research Diagnostics Inc, NJ, USA) and TNF-α from R&D Systems (Minneapolis, Minn, USA). Polyclonal anti-serum of hFAF1 was raised in mouse against recombinant human FAF1 (35).

Example 3.2 - Transient Transfection

HEK293T cells were transfected with expression plasmid using the calcium phosphate precipitation method. Cells were seeded in 10 cm plates a day before transfection at the density of 1.2 X 10⁶ cells and transiently transfected with 6-12 µg of expression plasmid by calcium phosphate method. Jurkat cells were transfected using nucleofactor solution provided by Amaxa apparatus (Amaxa, Cologne, Germany). For using the Amaxa system, 5 x 10⁶ Jurkat cells were harvested and then resuspended in 100 µL of specified electroporation buffer with 5 µg of plasmid DNA. Resuspended cells were transferred to a cuvette and nucleofected with an Amaxa Nucleofector apparatus.

Example 3.3 - Immunoprecipitation

Cells were disrupted with a lysis buffer containing protease inhibitors (50 mM Tris-Cl, pH 8.0, 150 mM NaCl, 2 mM EDTA, 0.5% NP-40, 1 mM PMSF, 0.5 mM DTT, 5 µg/mL aprotinin, 10 µg/mL pepstatin A, 10 µg/mL leupeptin) for 30 min on ice. The lysates were centrifuged at 12,000 rpm for 1 h and the supernatant was incubated for 3 h at 4°C with monoclonal anti-Flag M2 agarose crosslinking affinity beads (Sigma) at 4°C. For immunoprecipitating endogenous FAF1, cell lysates were incubated with mouse immunoglobulin G or polyclonal anti-FAF1 antibody and then with protein G beads. The immunoprecipitated beads were washed at least three times with 1 mL of lysis buffer containing 0.5% NP-40. The pellet was used for immunoblotting. For ubiquitin binding, cell pellet was lysed in 400 µL of hypotonic buffer containing protease inhibitors (10 mM HEPES, 1.5 mM MgCl₂, 10 mM KCl, 0.5 mM PMSF, 0.2 mM DTT, 5 µg/mL aprotinin, 10 µg/mL pepstatin A, 10 µg/mL leupeptin, pH 7.9) for 30 min on ice and centrifuged at 4,000 rpm for 15 min. The supernatant was incubated for 3 h at 4°C with monoclonal anti-Flag M2 agarose crosslinking affinity beads. Beads were washed three times with 1 mL of lysis buffer containing 0.5% NP-40.

Example 3.4 - Two-dimensional Gel Electrophoresis

The protein samples were mixed with a buffer containing 9.5 M urea, 2% Triton X-100, 5% β-mercaptoethanol, 1 mM PMSF, 5 µg/mL aprotinin, 10 µg/mL pepstatin A, 10 µg/mL leupeptin, 1 mM EDTA, 10 mM Na₃VO₄, 10 mM NaF for 30 min at room temperature and electrofocused in 7 cm Immobiline DryStrips (pH 4-7) with the Amersham IPGphor. The following focusing protocol was used: 50 µA per strip at 20°C; (1) rehydration for 16 h; (2) 500 V for 1 h (step and hold); (3) 1000 V for 1 h (step and hold); (4) 8000 V for 3 h (step and hold). After electrofocusing, the strips were shaken for 15 min with equilibration buffer (1.5 M Tris-Cl, pH 8.8, 6 M urea, 30% glycerol, 2% SDS, 10 mg/mL DTT) and loaded onto Bio-Rad mini 2D-gel SDS-PAGE.

Example 3.5 - In-Gel Digestion and Mass spectrometric analysis

The gel spots were excised with a scalpel, crushed and destained by washing with 25 mM ammonium bicarbonate/50% acetonitrile. The silver stained gel was destained by washing with 15 mM potassium ferricyanide/50 mM sodium thiosulfate, crushed, dehydrated by addition of acetonitrile, rehydrated by adding 10-20 µL of 25 mM ammonium bicarbonate with 10 µg/µL of sequencing grade trypsin (Promega), and incubated at 37°C for 12-15 h. Peptides were extracted three times with 30 µL of a solution containing 60% acetonitrile/0.1% trifluoroacetic acid and finally with 20 µL of acetonitrile. The extracts were pooled, and evaporated to dryness in a SpeedVac vacuum centrifuge. The dried peptides were mixed with saturated matrix solution α-cyano-4-hydroxycinnamic acid in 60% acetonitrile/0.1% trifluoroacetic acid and analyzed with MALDI-TOF MS (Voyager-DE STR; Applied Biosystems, Inc.) or ESI-q-TOF tandem mass spectrometry (Micromass/Waters Corp.) in conjunction with the MS-Fit program available on the World Wide Web site of the University of California at San Francisco (http://prospector.ucsf.edu/).

Example 3.6 - *In vitro* binding assay

GST-hFAF1 deletion mutant fusion proteins and GST-VCP were expressed in Escherichia coli BL21(DE3) cells and bound to glutathione sepharose 4B beads. The beads were washed three times with PBS containing 0.1% Triton X-100. GST-hFAF1 bound to glutathione-sepharose 4B beads was incubated with recombinant VCP. GST-VCP bound to glutathione-sepharose 4B beads was incubated with recombinant hFAF1 in binding buffer (50 mM Tris-Cl, pH 7.4, 150 mM NaCl, 1 mg/mL BSA) with rocking for 3 h at 4°C. For ubiquitin binding, GST-hFAF1 bound to beads was incubated with cytoplasmic extract from HEK293T cells or polyubiquitin chains (Ub₂₋₇) in binding buffer (50 mM Tris-Cl, pH 7.4, 150 mM NaCl, 1 mg/mL BSA) for 3 h at 4°C. The beads were washed five times with 1 mL of 0.1% triton X-100 in binding buffer and then resuspended in 2 x gel sample buffer.

Example 3.7 - Quantifying the degradation of ubiquitinated proteins

Proteolysis of ubiquitinated proteins was quantified using Ub^{G76V}-GFP as a substrate (8, 20). HEK293T cells were cotransfected with expression plasmid, 200 ng of Ub^{G76V}-GFP or EGFP-N1 vector and 1 µg of various truncated Flag-hFAF1 constructs in 35 mm dishes using the calcium phosphate precipitation method. After 24 h, the cells were washed with PBS and fluorescence of Ub^{G76V}-GFP in 10,000 cells was measured with a FACS flow cytometer (Beckton & Dickinson). Mean fluorescence intensity was calculated using Cellquest Software.

Example 3.8 - Cell Viability Assay

MTT conversion assay was employed assessing cell viability. Jurkat cells transiently transfected with expression plasmid using Amaxa Electroporator were seeded in 96 well plates at a density of 1 x 10⁵ cells/well. 10 µL of MTT (Sigma) was added, and then incubated for 2 h to allow metabolization of MTT to 3-[4,5-dimethyldiazol-2-yl]-2,5-diphenylformazan. The latter was solubilized with acidified isopropanol and absorbance was measured at 570 nm in a microplate reader.

**EXAMPLE 4** - RESULTS

Example 4.1 - hFAF1 interacts with VCP *in vitro* and *in* vivo

We attempted to identify the proteins interacting with hFAF1 as leads for understanding how hFAF1 functions. Flag-hFAF1 was transiently overexpressed in HEK293T cells. HEK293T cell lysates were immunoprecipitated with monoclonal anti-Flag M2 agarose crosslinking affinity beads and the immunoprecipitates analyzed by 2-dimensional gel electrophoresis. One fraction was transferred to PVDF membrane for western blot analysis and the other was silver stained for visualization. Flag-hFAF1 was detected at 80-85 kDa with silver staining and confirmed by western blotting (data not shown). In addition to hsp70 shown to interact with hFAF1 in an earlier study (17), we detected hsc70, and one new spot in the coprecipitates with Flag-tagged hFAF1, but not with control Flag. The new spot detected in the silver stained gel was subjected to in-gel digestion with trypsin and analysed using MALDI-TOF MS and ESI-q-TOF tandem MS. The protein interacting with hFAF1 was identified as valosin-containing protein (VCP, NCBI accession number, 6005942) by peptide mass finger printing using MALDI-TOF MS and confirmed by sequencing using ESI-q-TOF tandem MS (Fig. 11B).

To examine whether hFAF1 interacts with VCP directly, we performed reciprocal in vitro binding assays. GST-hFAF1 was immobilized to glutathione-sepharose beads and incubated with purified recombinant VCP. We observed direct interaction between GST-hFAF1 and VCP using anti-VCP antibody (Fig. 11C). In the reciprocal experiment, GST-VCP immobilized on glutathione beads was incubated with purified recombinant hFAF1 and hFAF1 bound to GST-VCP was detected with anti-FAF1 antibody (Fig. 11D).

In order to see if endogenous hFAF1 interacts with endogenous VCP in vivo, we performed immunoprecipitation studies with polyclonal anti-hFAF1 antibody in HEK293T cells and Jurkat cells. In both HEK293T and Jurkat cells, endogenous FAF1 coimmunoprecipitated with VCP as detected with anti-VCP antibody (Fig. 11E). This indicates that endogenous FAF1 interacts with VCP in vivo as well.

Example 4.2 - hFAF1 interacts with VCP via UBX domain

To characterize the domains of interaction between hFAF1 and VCP, various truncated Flag-hFAF1s were overexpressed in HEK293T cell and their interactions with endogenous VCP were examined. As shown in Fig. 12A, overexpressed truncated Flag-hFAF1s were immunoprecipitated with anti-Flag M2 agarose crosslinking affinity beads and immunoblotted using anti-Flag antibody (Fig. 12B) and anti-VCP antibody (Fig. 12C). As shown in Fig. 12, endogenous VCP binds to Flag-hFAF1 full form and to hFAF1 fragments containing amino acids 366-650 and 567-650. This suggests that VCP interacts with HEAF1 via UBX domain located in the amino acid sequence 567-650. These results are consistent with previous reports that p47, identified as a major protein in complex with VCP, and whose UBX domain is very similar in its structure to the UBX domain from hFAF1 (17, 37), interacts with VCP through the UBX domain. It appears that hFAF1 is another protein that interacts with VCP through its UBX domain.

Example 4.3 - hFAF1 interacts with VCP in cytosol

In previous reports, quail FAF1 (qFAF1) was shown to have a novel nuclear localization signal and to localize in the nucleus (11). We examined whether hFAF1 interacts with VCP in the cytosolic or nucleus fractions, and whether heat shock inducing ubiquitination can influence this interaction. HEK293T cells transfected with the Flag-hFAF1 were exposed to heat shock stress at 45°C for 45 min, and fractionated into cytosolic and nuclear fractions. As shown in Fig. 13A, both Flag-hFAF1 and VCP were present in cytosolic and nuclear fractions, but VCP was coimmunoprecipitated with Flag-hFAF1 only in the cytosolic fraction (Fig. 13B). The amount of VCP coimmunoprecipitating with Flag-hFAF1 significantly decreased from heat shock and increased during recovery, while the total amount of VCP in cytosol remained unchanged. This suggests that the interaction of hFAF1 with VCP occurred only in cytosol in a stress dependent manner (Fig. 13B) and may involved in the degradation of proteins.

Example 4.4 - hFAF1 binds to ubiquitinated substrates *in vitro* and *in vivo*

Because hFAF1 has multiple ubiquitin-related domains, and also interacts with VCP which is known to function in ubiquitin-proteasome pathway (21, 36), we investigated whether hFAF1 is involved in the ubiquitin-proteasome pathway. Various truncated Flag-hFAFls listed in Fig. 14A were overexpressed in HEK293T cell, immunoprecipitated with anti-Flag M2 agarose crosslinking affinity beads and immunoblotted with anti-ubiquitin antibodies. As shown in Fig. 14C, full Flag-hFAF1 bound to multiubiquitinated proteins. N-terminal fragments containing amino acids 1-81 seem to be the region of hFAF1 involved in binding to a degree equal to or better than that of full hFAF1. Flag-hFAF1[1-201] and Flag-hFAF1[1-345] also bound to multiubiquitinated proteins, but the binding was weaker than with the wild type.

To examine whether hFAF1 directly interacts with multiubiquitinated proteins, we performed in vitro binding assay. Various truncated GST-hFAF1s immobilized to glutathione-sepharose beads were incubated with polyubiquitin chains (Ub₂₋₇) and bindings were detected with immunoblotting using anti-ubiquitin antibody and synthetic Lys48-linked polyubiquitin chains (Ub₂₋₇) (Affinity Research), which mimic endogenous multiubiquitinated substrate. As shown in Fig. 15B, we found that full hFAF1 and its fragment containing amino acids 1-81 but not amino acid 82-650 bound to polyubiquitin chains (Ub₂₋₇) in vitro. In addition, GST-hFAF1 full form and GST-hFAF1[1-81] interacted with multiubiquitin chains but not to monoubiquitin. GST-hFAF1 [1-81] interacted with di-, tri-ubiquitin chains more strongly than GST-hFAF1 full form. However, both GST-hFAF1 full form and GST-hFAF1[1-81] have similar affinity to longer polyubiquitin chains. These results suggest that hFAF1 directly binds to multiubiquitinated proteins via amino acids 1-81 directly. Structural differences probably account for the differences in the binding affinities of hFAF1[1-81] and full hFAF1.

When the amino acid sequence of hFAF1[1-81] was aligned with different proteins using Jalview program, we observed homology to the UBA domain (ubiquitin associated domain), which is known to be involved in binding to multiubiquitinated proteins (Fig. 15C). N-terminal amino acid sequence of hFAF1 seems to be well conserved among species. It is identical to that of mouse FAF1 and is similar to that of p47 and Y33K, all of which contain C-terminal UBX domain. Rad23 and PLIC are proteins having central and C-terminal UBA domains, which bind to multiubiquitinated proteins via UBA domain (12, 37). UBA domain has conserved hydrophobic surface patch in solution structure and arrows in Fig. 15C, indicate the amino acid residues that have been shown in the surface of UBA domain (27). The hydrophobic surface patch of UBA domain is important because it interacts with the hydrophobic surface on the five-stranded β-sheet of ubiquitin. Although UBA domain of hFAF1 is not fully conserved with other UBA domains, the amino acid residues occupying the position on hydrophobic surface patch in three-dimensional structure are well conserved (Fig. 15C). Based on a combination of experimental results with homology search, it appears that hFAF1 belongs to a new family of proteins binding to multiubiquitinated proteins via N-terminal UBA domain.

Example 4.5 - hFAF1 binds to Lys48-linked and Lys63-linked ubiquitinated proteins via UBA domain

To determine which population of multiubiquitin chains can interact with hFAF1, we coupled GST-hFAF1 and GST-hFAF1[1-81] to glutathione-sepharose beads and incubated them with HEK293T cell lysates. Interaction was detected by western blot analysis using anti-ubiquitin antibodies (data not shown). High molecular weight multiubiquitinated proteins were analyzed by mass spectrometry (Fig. 16A). In ubiquitin-proteasome pathway, multiubiquitin are chained through Lys48-Gly76 isopeptide bonds, with the proximal Ub linked to substrate lysine residue (30). Tryptic digestion of ubiquitin-conjugated protein produced a signature peptide at the ubiquitination site containing a two-residue remnant (GlyGly). This was derived from the C-terminus of ubiquitin, covalently attached to lysine residue of target protein via an isopeptide bond. This signature peptide has a mass shift 114.1 Da at the lysine residue as well as a missed proteolytic cleavage because trypsin cannot break at peptide bonds involving modified lysines (30). Using MALDI-TOF MS, we identified high molecular interacting proteins of hFAF1 as multiubiquitin chain (NCBI accession #136670). In addition, we detected two increased 114.1 Da peaks (m/z, 1460.7870; m/z, 2244.1814) resulting from amino acid residues 43-54 and 55-72 respectively (Fig. 16B, Table 2). To verify that the 114.1 Da mass shifts occur because of isopeptides from ubiquitin chain, we sequenced each peak using ESI-q-TOF tandem mass spectrometry and identified them as Lys48-GlyGly (Fig. 16C) and Lys63-GlyGly isopeptides respectively (data not shown). These results demonstrated that hFAF1 interacts with Lys48-linked and Lys63-linked multiubiquitin chains.

**Table 2. Expected and observed peptide fragments obtained from trypsin cleavage of ubiquitin associated with GST-hFAF1.**

| Tryptic peptide | Expected m/z | Observed m/z | sequence | modification |
|---|---|---|---|---|
| 12-27 | 1787.9278 | 1787.9066 | | |
| 30-42 | 1523.7818 | 1523.7634 | | |
| 34-42 | 1039.5172 | 1039.5037 | | |
| 43-54 | 1346.7432 | 1460.7870 | LEFAGK(- GG)QLEDGR | Lys-GlyGly |
| 55-72 | 2130.1559 | 2130.1343 | | |
| 55-72 | 2130.1559 | 2244.1814 | TLSDYNQK(- GG)ESTLHLVLR | Lys-GlyGly |
| 64-72 | 1067.6213 | 1067.6204 | | |

Example 4.6 - hFAF1 inhibits degradation of ubiquitin-dependent substrates,

Fig. 17 shows that overexpression of Flag-hFAF1 and Flag-hFAF1[1-81] caused the accumulation of multiubiquitinated proteins in contrast to the overexpression of pFlag-CMV vector and Flag-hFAF1[82-650]. We hypothesize that the accumulation of the intracellular multiubiquitinated proteins following overexpression of hFAF1 occurred because hFAF1 complexed with them and prevented or inhibited their proteolytic degradation. To test this hypothesis, we decided to examine whether hFAF1 influence the proteolysis of ubiquitinated proteins. For this, we employed the GFP based proteasome substrate, LTb^{G76V}-GFP, used by Dantuma et al. for quantification of ubiquitin-proteasome-dependent proteolysis in living cells (8, 20). We first confirmed that this system works under our experimental conditions (Fig.' 21). We then transiently co-transfected HEK293T cells with 200 ng of Ub^{G76V}-GFP and 1 µg of Flag-hFAF1 or Flag-VCP and measured GFP fluorescence intensity by flow cytometry after proteasome degradation at 24 h after transfection.

Overexpression of Flag-hFAF1 significantly reduced the Ub^{G76V}-GFP degradation detected with western analysis (Fig. 18A) and raised the mean fluorescence intensity two fold compared to overexpression of pFlag-CMV vector (Fig. 18B). To exclude the possibility that the difference of mean fluorescence intensity may be due to transfection efficiency of Ub^{G76V}-GFP, we performed the same experiment using EGFP-N1 vector. HEK293T cells were transiently co-transfected with EGFP-N1 vector and Flag-hFAF1 or Flag-VCP. At 24 h after transfection, GFP protein level was determined in western analysis using GFP antibody and normalized with tubulin. As shown in Fig. 18A, GFP intensity based on the transfection efficiency remained unchanged confirming that increase in hFAF1 was specifically responsible for the decreased degradation of Ub^{G76V}-GFP reporter in proteasome.

Using flow cytometry and western blotting, we found that overexpression of Flag-VCP decreased the level Ub^{G76V}-GFP reporter (Fig. 18). This was as expected because VCP is known to recruit ubiquitinated proteins and to promote their degradation in proteasome (6, 7). When cotransfected with Flag-hFAF1 and Flag-VCP, the fluorescence level of Ub^{G76V}-GFP reporter was in between the fluorescence level of VCP and hFAF1. There are two possible explanations for this. One is that this observed fluorescence is the sum of fluorescence levels of Flag-hFAF1 and Flag-VCP. The other explanation is that VCP might negatively regulate hFAF1 and cause the observed fluorescence. We attempted to determine which of these possibilities is correct in fact occurring and which domain of hFAF1 is necessary for stabilizing proteasomal substrates by inhibiting their degradation. For this, HEK293T cells were cotransfected with Ub^{G76V}-GFP reporter and various truncated forms of Flag-hFAF1. Using flow cytometry and western blotting, we found that overexpression of full Flag-hFAF1 increased the level of Ub^{G76V}-GFP and that deletion of UBA domain of hFAF1[82-650] had no effect (Fig. 18C, 18D). Flag-hFAF1[1-81] and Flag-hFAF1[1-345] containing UBA domain, but not VCP binding domain, increased fluorescence level significantly over that of intact hFAF1 (Fig. 18C and 18D). These results indicate that UBA domain of hFAF1 is both necessary and sufficient for causing the accumulation of the ubiquitinated proteasomal substrates, and that VCP, interacting with hFAF1 via C-terminus, inhibits this accumulation caused by hFAF1.

Example 4.7 - hFAF1 interferes with the proteolytic degradation of endogenous ubiquitinated substrate

Since hFAF1 inhibited degradation of Ub^{G76V}-GFP, we examined whether hFAF1 can also affect degradation of endogenous ubiquitinated substrates. We selected IκBα as endogenous substrate, because IκBα is known to be degraded by ubiquitin-proteasome pathway (39) and also, VCP is associated with ubiquitinated IκBα when recruited to proteasome (7). HEK293T cells transfected with Flag-hFAF1 or its truncates were exposed to tumor necrosis factor α (TNF-α, 10 ng/mL) for 5, 10, 15, and 20 min at 24 h after transfection. As shown in Fig. 19, total cell lysates were immunoblotted using IκBα antibody and TNF-α lead to the rapid degradation of IκBα depending on the length of exposure. In untreated cells, IκBα content was almost the same irrespective of overexpression of DNA. However, overexpression of Flag-hFAF1 and Flag-hFAF1[1-8] interfered with IκBα degradation, whereas Flag vector and Flag-hFAF1[366-650] did not (Fig. 19A). IκBα content remained unchanged up to 15 min during which Flag-hFAF1 was overexpressed, and up to 20 min during which Flag-hFAF1[1-81] was overexpressed. Protein levels, normalized with tubulin, show that there was no degradation by TNF-α treatment (Fig. 19B). Consistent with the results using Ub^{G76V}-GFP reporter, the effect of Flag-hFAF1[1-81] is stronger than that of Flag-hFAF1. It is possible that Flag-hFAF1[1-81] has an enhanced effect because VCP cannot bind to it. Besides, absence of any difference in the total IκBα content suggests that hFAF1 affects only ubiquitinated protein. Taken together, these results indicate that hFAF1 inhibits the degradation of artificial as well as endogenous ubiquitinated proteins.

Example 4.8 - Cell death is induced by overexpression of hFAF1 and requires the UBA domain

It has been reported that hFAF1 overexpression without any treatment can initiate apoptosis in BOSC23 cells, and that hFAF1 interacts with the members of the Fas-death inducing signaling complex (Fas-DISC) in Jurkat cells (34, 35). However, the relationship of ubiquitination and cell death by hFAF1 is not clear. We therefore, examined whether accumulation of ubiquitinated proteins caused by hFAF1 affects hFAF1-induced cell death. We used Jurkat cells in this study because earlier studies employed these cells, among others, to study cell death caused by FAF1 (34, 35). Jurkat cells were transiently transfected with Flag-hFAF1 or truncates using Amaxa electroporator and cell viability was assessed as described. Overexpression of Flag-hFAF1 induced cell death as previously reported (34, 35) and Flag-hFAF1 [1-81] containing UBA domain significantly increased cell death (Fig. 20A). The effect on cell death of hFAF1 [1-81] which as only the UBA domain, is stronger than that of full hFAF1, consistent with GFP-reporter experiments. In the case of Flag-hFAF1[366-650], cell viability rather increased compared to controls. To confirm the cell death effect of UBA domain, we transiently transfected Jurkat cells with various amounts of Flag- hFAF1 [1-81] using Amaxa electroporator and assessed the cell viability by MTT assay. Cell death was induced by Flag-hFAF1[1-81], in a manner, dependent on transfected DNA concentration (Fig. 20B), suggesting that UBA domain of hFAF1 induces cell death by causing the accumulation of ubiquitinated proteins (Fig. 17 and Fig. 20).

**EXAMPLE 5** - EXPERIMENTAL PROCEDURES RELATED TO DIFFERENTIAL EXPRESSION OF FAF1 IN NORMAL VERSUS OVARIAN TUMOR

A normal or tumorous tissue sample to be assayed was washed 3 times in cold 1x PBS. The sample was cut into small pieces with sterile lancet Red blood cell lysis buffer was added to the sample to remove red blood cells. Lysis buffer (10mM Tris acetate, 10mM NaCl, 0.1mM EDTA, protease inhibitors) in an amount of 3x the sample volume was added and the sample homogenized and centrifuged at 1200 rpm at 4 °C, for about 30 minutes and the cytosol obtained.

Presence of various proteins in the normal and tumor cells was assayed. Western blot assay on various proteins was carried out using standard assay protocol using antibodies against the various proteins. Assayed proteins include actin, α-tubulin, FAF1, Hsp70, Hsp27, VCP, ITSN, UCH L1, SUMO, Secretagogen, Nm23-poly Ab, Nm23-mono-Ab, and GAPDH (Figs. 22 and 23).

The results show that FAF1 is differentially expressed in normal cells. FAF1 is less expressed in ovarian tumor cells.

### REFERENCES

**1.** Bays, N. W. and Hampton, R. Y. 2002. Cdc48-ufd1-np14: stuck in the middle with Ub. Curr. Biol. 12: R366-R371.
**2.** Becker, K., Schneider, P., Hofmann, K., Mattmann, C. and Tschopp, J. 1997. Interaction of Fas(Apo-1/CD95) with proteins implicated in the ubiquitination pathway. FEBS Lett. 412: 102-106.
**3.** Buchberger, A., Howard, M. J., Proctor, M. and Bycroft, M. 2001. The UBX domain: a widespread ubiquitin-like module. J. Mol. Biol. 307: 17-24.
**4.** Chen, L. and Madura, K. 2002. Rad23 promotes the targeting of proteolytic substrates to the proteasome. Mol. Cell. Biol. 22: 4902-4913.
**5.** Chu, K., Niu, X. and Williams, L. T. A. 1995. Fas-associated protein factor, FAF1, potentiates Fas-mediated apoptosis. Proc. Natl. Acad. Sci. USA. 92: 11894-11898.
**6.** Dai, R. M. and Li C. C. H. 2001. Valosin-containing protein is a multiubiquitin chain-targeting factor required in ubiquitin-proteasome degradation. Nat. Cell Biol. 3: 740-744.
**7.** Dai, R. M., Chen, E., Longo, D. L., Gorbe, C. M. and Li C. C. H. 1998. Involvement of valosin-containing protein, an ATPase copurified with IκBα and 26S proteasome, in ubiquitin-proteasome mediated degradation of IκBα. J. Biol. Chem. 273: 3562-3573.

## Claims

1. A polypeptide fragment of Fas associated Factor 1, which binds to ubiquinated protein.

2. The fragment according to claim 1, which is ubiquitin associated (UBA) domain of Fas associated Factor 1.

3. The fragment according to claim 2, wherein the Fas associated Factor 1 is human Fas associated Factor 1.

4. The fragment of Fas associated Factor 1 according to claim 3, which is from about amino acid position 1 to about 81.

5. A method for preventing degradation of an ubiquinated protein comprising contacting the ubiquinated protein with the fragment according to claim 1.

6. The method according to claim 5, wherein the fragment is ubiquitin associated (UBA) domain of Fas associated Factor 1.
